# EUROPEAN PATENT SPECIFICATION

(11) **EP 4 548 950 B1**
(45) Date of publication and mention of the grant of the patent: **27.05.2026**
(21) Application number: 25164998.4
(22) Date of filing: 08.10.2021
(51) Int. Cl.: A61C 7/08, A61F 5/56, A61B 5/08, A61B 5/113, A61B 5/00, A61M 16/00

(54) **AUTOMATIC ORAL APPLIANCE ADJUSTMENT**
AUTOMATISCHE ANPASSUNG EINER ORALEN VORRICHTUNG
RÉGLAGE AUTOMATIQUE D'APPAREIL BUCCAL

(30) Priority: 09.10.2020 US 202063090004 P
(43) Date of publication of application: 07.05.2025
(62) Divisional of application: 21791476.1
(73) Proprietor: ResMed Sensor Technologies Limited, Sandyford D18 T6T7 Dublin (IE)
(72) Inventor: SHOULDICE, Redmond, Dublin, D18 T6T7 (IE); WREN, Michael, Dublin, D18 T6T7 (IE)
(74) Representative: Mathys & Squire

(56) References cited:
- WO-A1-2016/157129
- WO-A1-2017/132726
- US-A1- 2016 199 215

## Description

### TECHNICAL FIELD

The present disclosure relates to treatment of sleep conditions generally and more specifically to oral appliances with automatic adjustments for treatment of sleep conditions.

### BACKGROUND

Many individuals suffer from sleep-related disorders associated with one or more events that occur during sleep, such as, for example, snoring, an apnea, a hypopnea, a restless leg, a sleeping disorder, choking, an increased heart rate, labored breathing, an asthma attack, an epileptic episode, a seizure, or any combination thereof. These individuals are often treated using one or more medical devices to improve sleep and reduce the likelihood of events occurring during sleep. While some such medical devices rely on providing positive airway pressure to the individual, some medical devices for the treatment of sleep conditions include oral appliances (e.g., a dental appliance or a mandibular repositioning device) that can be worn by the individual during sleep.

Oral appliance therapy can help prevent the collapse of the tongue and soft tissues in the back of the throat by supporting the jaw (mandible) in a forward position, keeping the user's airway open during sleep. In some cases, oral appliance therapy may be especially useful for individuals with lower body mass index (BMI) and lower apnea hypopnea index (AHI). Oral appliance therapy can be especially effective for positional (e.g., supine dependent) obstructive sleep apnea.

When an individual desires to use an oral appliance to treat a sleep condition, the oral appliance is generally fitted to the user with the assistance of a medical professional. Various adjustments can be made to the oral appliance to provide the most effective fit and function. In some cases, several visits may be required to achieve desired fit and function. At each visit, the medical professional may need to make small adjustments to the oral appliance to ensure it is operating as desired. Because visits to a medical professional may be separated by one or more days, current oral appliances may not be able to be properly adjusted between such visits. Unfortunately, because of the need for multiple visits to a medical professional and the chance that the oral appliance may not work as intended if not properly adjusted, some individuals may forgo using the oral appliance and thus not comply with their prescribed therapy, which can be detrimental to the individual.

WO 2016/157129 discloses systems and methods for setting one or more parameters for a multi-testperiod titration for oral appliance therapy.

### SUMMARY

The invention is as defined in the appended claims.

According to some implementations of the present disclosure, a method for adjusting an oral appliance includes receiving sensor data from one or more sensors external to a user using an oral appliance for the treatment of a sleep condition. The method also includes automatically determining an adjustment associated with the oral appliance based on the sensor data. The method also includes facilitating applying the determined adjustment to the oral appliance in response to automatically determining the adjustment.

The invention relates to a system comprising an oral appliance (122) configured to be used by a user for treatment of a sleep condition; one or more sensor (130); an oral appliance receptacle (126) configured to receive the oral appliance (122) when not used by the user; a control system (110) including one or more processors (112); and a memory (114), the control system (110) coupled to the memory (114), the memory (114) having stored thereon machine readable instructions which, when executed on the one or more processors (112), causes the one or more processors (112) to perform operations including: receiving sensor data from the one or more sensors (130); automatically determining an adjustment associated with the oral appliance (122) based on the sensor data; and facilitating applying the determined adjustment to the oral appliance (122) in response to automatically determining the adjustment, wherein facilitating applying the determined adjustment includes actuating one or more actuators (128) of the oral appliance receptacle (126) to apply the determined adjustment to the oral appliance (122).

The above summary is not intended to represent each implementation or every aspect of the present disclosure. Additional features and benefits of the present disclosure are apparent from the detailed description and figures set forth below.

### BRIEF DESCRIPTION OF THE DRAWINGS

FIG. 1 is a functional block diagram of a system for automatically adjusting an oral appliance, according to certain aspects of the present disclosure.
FIG. 2 is a perspective view of the system of FIG. 1, a user, and a bed partner, according to certain aspects of the present disclosure.
FIG. 3 illustrates an example timeline for a sleep session, according to certain aspects of the present disclosure.
FIG. 4 illustrates an example hypnogram associated with the sleep session of FIG. 3, according to certain aspects of the present disclosure.
FIG. 5 is a flowchart depicting a process for automatically adjusting an oral appliance, according to certain aspects of the present disclosure.

While the present disclosure is susceptible to various modifications and alternative forms, specific implementations and embodiments thereof have been shown by way of example in the drawings and will herein be described in detail.

### DETAILED DESCRIPTION

Certain aspects and features of the present disclosure relate to automatic adjustment of an oral appliance, such as a mandibular repositioning device. Sensor data can be received from one or more sensors external to a user using the oral appliance. An adjustment associated with the oral appliance is determined using the sensor data, and an action can be taken to facilitate applying the determined adjustment. Such actions can include transmitting a signal to the oral appliance to effect the adjustment (e.g., using an actuator or onboard electrical stimulator), presenting adjustment parameters to help the user manually make the adjustment, activating actuators in an oral appliance storage receptacle the next time the oral appliance is stored, or other such actions. Adjustments can be made dynamically in real-time or asynchronously (e.g., between sleep sessions).

Oral appliance therapy can include the use of an oral appliance system to treat a sleep condition. Examples of sleep conditions are described herein. A sleep condition can include any known or unknown condition that affects a user during sleep. A sleep condition can include any known or unknown condition that affects a user's ability to sleep and/or quality of sleep. A sleep condition can include any known or unknown condition that affects a user's ability to breath during sleep.

An oral appliance system can include an oral appliance that can be used by a user (e.g., worn in a mouth (e.g., an oral cavity) of the user). An oral appliance system can optionally include an oral appliance receptacle for storing the oral appliance when not in use. The oral appliance receptacle can be a simple container or receptacle for receiving the oral appliance, or can include additional components for charging, communicating with, and/or adjusting the oral appliance.

The oral appliance is generally a fully removable oral appliance (e.g., fully removable from the oral cavity of a user), although that need not always be the case. The oral appliance can include one or more adjustments (e.g., adjustable aspects of the oral appliance), such as an adjustable coupling of a mandibular repositioning device. In such an example, the mandibular repositioning device may include an upper tray and a lower tray coupled by an adjustable coupling. The upper tray is typically configured to engage the upper jaw and/or teeth of the upper jaw, while the lower tray is configured to engage the lower jaw and/or teeth of the lower jaw. The adjustable coupling can be adjusted to control the relative position of the upper tray with respect to the lower tray, and thus the relative position of the upper and lower jaws. For example, the adjustable coupling can include an adjustable-length strut rotatably coupled to the upper tray and lower tray at fixed points. In another example, the adjustable coupling can include a fixed-length strut rotatable coupled to the upper tray and lower tray at adjustable points. In some cases, the adjustable coupling can be adjusted to control the resistance and/or biasing force associated with movement of the upper tray with respect to the lower tray, which can correlate to the amount of force a user needs to use to separate and bring together their teeth. Other adjustments can be made.

In some cases, adjustment of the oral appliance can be manually effected, with or without the aid of tools. For example, the oral appliance might be adjustable entirely by hand or might be adjustable with use of a screwdriver or other tool. In such cases, certain aspects of the present disclosure can include presenting a display to a user or other individual (e.g., medical professional) with adjustment parameters. These adjustment parameters can indicate how the oral appliance is to be adjusted. For example, an adjustment parameter can include instructions to shorten an adjustable-length strut by 1mm, 2mm, 3mm, or the like.

In some cases, adjustment of the oral appliance can be automatically effected at the oral appliance. Automatically effecting adjustment at the oral appliance can include adjusting a software setting of the oral appliance (e.g., adjusting the driving settings for an electrical stimulator in the oral appliance) or manipulating one or more physical adjustments of the oral appliance (e.g., driving an actuator in the oral appliance to change the relative position of an upper tray with respect to a lower tray). In some cases, the oral appliance can include an actuator capable of manipulating the one or more adjustments of the oral appliance. Any suitable actuator can be used, such as a electromechanical actuator, a piezoelectric actuator, a linear actuator, a rotational actuator, a motor, a servo, a pump, a screw drive, an electromagnetic actuator, or the like.

Adjustment of the oral appliance at the oral appliance can include transmitting a signal to the oral appliance from a control system. Once received by the oral appliance, the signal can effect adjustment of the oral appliance. In some cases, the signal can be sent while the oral appliance is in use, thus permitting dynamic, real-time adjustment of the oral appliance. In some cases, however, the signal can be sent to the oral appliance when the oral appliance is not in use (e.g., when the oral appliance is in an oral appliance receptacle).

In some cases, adjustment of the oral appliance can be automatically effected using an external actuator, such as an actuator within an oral appliance receptacle. In such cases, one or more sensors can detect that the oral appliance is received by the oral appliance receptacle, then one or more actuators of the oral appliance receptacle can adjust the one or more adjustments of the oral appliance. For example, a mandibular repositioning device can be placed in an associated receptacle, after which an actuator of the receptacle can manipulate an adjustable coupling of the mandibular repositioning device to change the relative position of the upper tray with respect to the lower tray.

The type and amount of adjustment to be made can be automatically determined by a system. The system can receive sensor data from one or more sensors, then use the sensor data to determine the adjustment to be made. In some cases, the system can also receive stored data, such as historical sensor data, historical adjustments, preset settings or routines, or the like. The system can operate using one or more algorithms, machine learning models (e.g., neural networks), or other techniques to interpret the incoming data (e.g., sensor data and stored data) and determine the desired adjustment. The determined adjustment can be designed to improve treatment of a user's sleep condition, reduce discomfort, reduce or minimize sleep events (e.g., apnea events), or otherwise improve the sleep of the user.

In some cases, the system determines adjustments dynamically (e.g., in real-time), such as using real-time sensor data. In some cases, the system can determine adjustments asynchronously, such as using stored sensor data. In some cases, the system can cause the adjustment to be applied dynamically (e.g., in real-time or near real-time), such as while the user is sleeping. In other cases, the system can cause the adjustment to be applied asynchronously, such as while the user is not sleeping. In some cases, the system can control adjustment of the oral appliance to move in small amounts over time to help a user become accustomed to the adjustment. Such small adjustments over time can be over a single sleep session (e.g., to slowly move the oral appliance from a comfort-prioritized state to a treatment-prioritized state) or over multiple sleep sessions (e.g., to slowly work a new oral appliance user up from a minimum treatment level to a desired treatment level over the course of multiple days).

The sensor data used by the system can include sensor data from one or more sensors across one or more devices. For example, sensor data can include sensor data from one or more sensors in the oral appliance, from one or more sensors in an oral appliance receptacle, from one or more sensors in a user device (e.g., a smartphone or computer), or from one or more sensors in another external device. In some cases, the sensor data is from one or more sensors external to the user. In some cases, the system makes use of data associated with a sleep session in which the user is using the oral appliance. In some cases, the system can make use of data associated with a sleep session in which the user is not using the oral appliance. In such cases, the system can be used to determine differences associated with use of the oral appliance versus non-use of the oral application such as, for example, differences associated with the user's sleep condition, co-morbidities experienced by the user (e.g., hypertension, diabetes, insomnia, and the like), occurrence of sleep events (e.g., apnea events), quality of sleep, or any combination thereof.

In some cases, determining the adjustment can include analyzing the sensor data to determine a sleep state or sleep stage associated with the user. Examples of the determination of sleep state and/or sleep stage is further described in, for example, WO 2014/047310, US 2014/0088373, WO 2015/006364, WO 2017/132726, WO 2019/122413, and WO 2019/122414. In some cases, certain adjustments can be made depending on the sleep state or sleep stage of the user. For example, the oral appliance may be initially adjusted to a comfort-prioritized state for sleep onset (e.g., while the user is awake and starting to fall asleep), but once it is determined that the user is in a sleep state or sleep stage after sleep onset, the system may adjust the oral appliance to a treatment-prioritized state. In the comfort-prioritized state, comfort may be prioritized over treatment efficacy, and the oral appliance may be more comfortable to the user, but may not provide as effective treatment for the sleep condition. In the treatment-prioritized state, treatment efficacy may be prioritized over comfort, and the oral appliance may be more effective in treating the sleep condition, but may not be as comfortable. Since the user is already asleep when the adjustment is made to put the oral appliance into the treatment-prioritized state, the user may not notice the reduced comfort, but may benefit from the increased treatment efficacy.

In some cases, determining the adjustment can include analyzing the sensor data to identify and/or predict an event, such as an apnea event. In cases where an event is identified or detected, the system may make adjustments to put the oral appliance into a post-event state. In the post-event state, the oral appliance may apply increased correction designed to counteract, reduce, minimize, and/or eliminate the event (e.g., the apnea event) or occurrence of a subsequent event. In cases where a future event is predicted based on sensor data, the system may make adjustments to put the oral appliance into a pre-event state. In the pre-event state, which can be an enhanced state (e.g., a state designed to be more therapeutically effective), the oral appliance may apply certain corrections to the user that are designed to avoid or minimize the future event. For example, moving into a pre-event state can include adjusting the oral appliance to improve therapeutic effectiveness for the user, possibly at the cost of decreasing comfort to the user.

In some cases, an oral appliance can be used to drive other equipment, such as an implantable treatment device. In such cases, adjustments to the oral appliance can include adjustments to software settings to adjust how the oral appliance controls the implantable treatment device.

In some cases, the system can interact with other external devices associated with a user's sleep session. In an example, a pillow, blanket, and/or mattress can include one or more inflatable bladders that can be controlled by the system. In this example, if the system detects that the user is sleeping while using an oral appliance, the system can control the one or more inflatable bladders to urge the user into a desired position. For example, if the system detects the user is sleeping with a mandibular repositioning device in place, the system may control one or more inflatable bladders to urge the user into a side sleeping position. In some cases, the user may be urged into other sleeping positions, such as a supine sleeping position. Other external devices can be used. In some cases, the system can adjust the oral appliance and one or more external devices together, such as to try and achieve a desired result (e.g., minimization, reduction, or elimination of an event or series of events). For example, if adjustment of the oral appliance is insufficient to achieve the desired result (e.g., if a determined adjustment would be beyond a threshold, such as a user-comfort threshold or a system-capability threshold), the system may additionally control an external device to achieve the desired result. In other examples, if the system detects that the user had entered bed and/or is sleeping while not using an oral appliance (e.g., if the user forgets to put the oral appliance into their mouth before going to sleep), the system may alert the user, such as with a notification via a user device, or the system may control an external device to compensate for the absence of the oral appliance and to achieve the desired therapeutic result, such as by urging the user to a side or supine sleeping position.

In some cases, the process of receiving sensor data, determining an adjustment, and applying the adjustment can create a feedback loop capable of controlling aspects of a user's sleep session to effectively treat a sleep condition.

In some cases, automatic adjustment of the oral appliance occurs continuously while the user is using the oral appliance or while the user is determined to be asleep while using the oral appliance. In some cases, automatic adjustment occurs occasionally (e.g., once every hour, every few hours, every day, every few days, every week, every few weeks, every month, every few months, every year, or every few years). In some cases, automatic adjustment occurs only a set number of times (e.g., once) per sleep session or per inserting the oral appliance. In some cases, automatic adjustment occurs only after being manually initiated, such as by pressing a button or control on an external device associated with starting automatic adjustment of the oral appliance. In some cases, automatic adjustment occurs upon determining, through one or more sensors, that a new oral appliance is being used.

These illustrative examples are given to introduce the reader to the general subject matter discussed here and are not intended to limit the scope of the disclosed concepts. The following sections describe various additional features and examples with reference to the drawings in which like numerals indicate like elements, and directional descriptions are used to describe the illustrative embodiments but, like the illustrative embodiments, should not be used to limit the present disclosure. The elements included in the illustrations herein may not be drawn to scale.

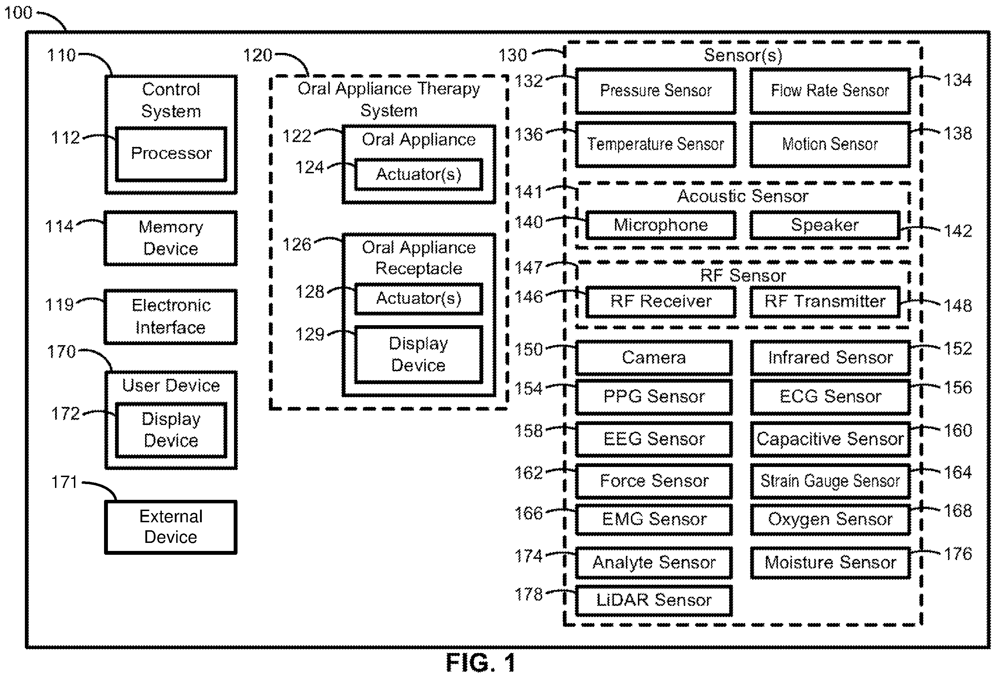

Referring to FIG. 1, a system 100 includes a control system 110, an oral appliance therapy system 120, one or more sensors 130, a user device 170, and an external device 171. As described herein, the system 100 generally can be used to provide oral appliance therapy to a user as well as automatically make adjustments to that therapy via adjustments to the oral appliance 122.

The control system 110 includes one or more processors 112 (hereinafter, processor 112). The control system 110 is generally used to control (e.g., actuate) the various components of the system 100 and/or analyze data obtained and/or generated by the components of the system 100. The processor 112 can be a general or special purpose processor or microprocessor. While one processor 112 is shown in FIG. 1, the control system 110 can include any suitable number of processors (e.g., one processor, two processors, five processors, ten processors, etc.) that can be in a single housing, or located remotely from each other. The control system 110 can be coupled to and/or positioned within, for example, a housing of the user device 170, a housing of the external device 171, a portion (e.g., a housing) of the oral appliance therapy system 120 (e.g., the oral appliance 122 or oral appliance receptacle 126), and/or within a housing of one or more of the sensors 130. The control system 110 can be centralized (within one such housing) or decentralized (within two or more of such housings, which are physically distinct). In such implementations including two or more housings containing the control system 110, such housings can be located proximately and/or remotely from each other.

The memory device 114 stores machine-readable instructions that are executable by the processor 112 of the control system 110. The memory device 114 can be any suitable computer readable storage device or media, such as, for example, a random or serial access memory device, a hard drive, a solid state drive, a flash memory device, etc. While one memory device 114 is shown in FIG. 1, the system 100 can include any suitable number of memory devices 114 (e.g., one memory device, two memory devices, five memory devices, ten memory devices, etc.). The memory device 114 can be coupled to and/or positioned within a housing of the oral appliance therapy system 120 (e.g., within a housing of an oral appliance receptacle 126), within a housing of the user device 170, within a housing of the external device 171, within a housing of one or more of the sensors 130, or any combination thereof. Like the control system 110, the memory device 114 can be centralized (within one such housing) or decentralized (within two or more of such housings, which are physically distinct).

The electronic interface 119 is configured to receive data (e.g., physiological data, environmental data, audio data, movement data, and the like) from the one or more sensors 130 such that the data can be stored in the memory device 114 and/or analyzed by the processor 112 of the control system 110. The electronic interface 119 can communicate with the one or more sensors 130 using a wired connection or a wireless connection (e.g., using an RF communication protocol, a WiFi communication protocol, a Bluetooth communication protocol, over a cellular network, etc.). The electronic interface 119 can include an antenna, a receiver (e.g., an RF receiver), a transmitter (e.g., an RF transmitter), a transceiver, or any combination thereof. The electronic interface 119 can also include one more processors and/or one more memory devices that are the same as, or similar to, the processor 112 and the memory device 114 described herein. In some implementations, the electronic interface 119 is coupled to or integrated in the user device 170. In some implementations, the electronic interface 119 is coupled to or integrated in the external device 171. In other implementations, the electronic interface 119 is coupled to or integrated (e.g., in a housing) with the control system 110 and/or the memory device 114. In some cases, multiple electronic interfaces 119 can be used across multiple components of system 100 (e.g., oral appliance 122, oral appliance receptacle 126, user device 170, and external device 171).

The oral appliance therapy system 120 includes an oral appliance 122 (e.g., a mandibular repositioning device) and optionally an oral appliance receptacle 126. The oral appliance 122 can be a device insertable into an oral cavity of a user to apply oral appliance therapy. The oral appliance receptacle 126 can be any container or suitable receptacle for receiving the oral appliance 122, such as for storage. The oral appliance receptacle 126 can include a receiving space for receiving the oral appliance 122. Oral appliance therapy refers to the use of an oral appliance to treat a sleep condition, such as obstructive sleep apnea. Oral appliance therapy can include applying force to one or more elements of a user's oral cavity (e.g., the mandible) to treat the sleep condition, such as described herein.

The oral appliance 122 can include one or more adjustments, also known as adjustable aspects. In some cases, the oral appliance 122 optionally includes one or more actuators 124 capable of inducing changes in the one or more adjustments (e.g., adjusting the oral appliance). In some cases, the oral appliance receptacle 126 optionally includes one or more actuators 128 capable of inducing changes in the one or more adjustments of the oral appliance (e.g., capable of adjusting the oral appliance) when the oral appliance is received by the oral appliance receptacle. The one or more actuators 128 can be positioned to manipulate an oral appliance 122 when received in the receiving space of the oral appliance receptacle 126.

The oral appliance therapy system 120 is generally used to treat individuals suffering from one or more sleep-related respiratory disorders (e.g., obstructive sleep apnea).

In some cases, the oral appliance receptacle 126 can include a display device 129. The display device 129 is generally used to display image(s) including still images, video images, or both and/or information regarding the oral appliance 122. For example, the display device 129 can provide information regarding the status of the oral appliance 122 (e.g., whether or not the oral appliance 122 is in use or has been cleaned since last use, current temperature of the oral appliance 122, or other information associated with the oral appliance 122), current settings of one or more adjustable aspects of the oral appliance 122, and/or other information (e.g., information about the most recent sleep session, the current date/time, personal information for the user, and the like). In some implementations, the display device 129 acts as a human-machine interface (HMI) that includes a graphic user interface (GUI) configured to display the image(s) as an input interface. The display device 129 can be an LED display, an OLED display, an LCD display, or the like. The input interface can be, for example, a touchscreen or touch-sensitive substrate, a mouse, a keyboard, or any sensor system configured to sense inputs made by a human user interacting with the oral appliance receptacle 126.

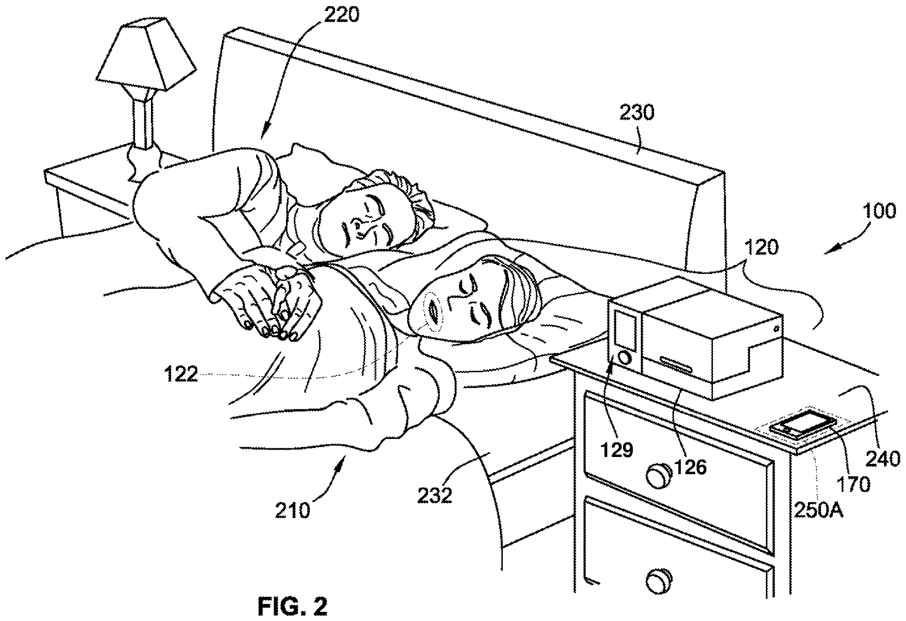

Referring to FIG. 2, a portion of the system 100 of FIG. 1, according to some implementations, is illustrated. A user 210 of the oral appliance therapy system 120 and a bed partner 220 are located in a bed 230 and are laying on a mattress 232. The oral appliance 122 (e.g., a mandibular repositioning device) can be used by the user 210 (e.g., worn within the oral cavity) during a sleep session.

In some cases, the oral appliance therapy system 120 can include an oral appliance receptacle 126, which can be positioned on a nightstand 240 that is directly adjacent to the bed 230 as shown in FIG. 2, or more generally, on any surface or structure that is generally adjacent to the bed 230 and/or the user 210. In some cases, the oral appliance therapy system 120 can be placed in a location not adjacent the bed 230 and/or user 210, such as in a bathroom or other location.

Referring to back to FIG. 1, the one or more sensors 130 of the system 100 include a pressure sensor 132, a flow rate sensor 134, temperature sensor 136, a motion sensor 138, a microphone 140, a speaker 142, a radio-frequency (RF) receiver 146, a RF transmitter 148, a camera 150, an infrared sensor 152, a photoplethysmogram (PPG) sensor 154, an electrocardiogram (ECG) sensor 156, an electroencephalography (EEG) sensor 158, a capacitive sensor 160, a force sensor 162, a strain gauge sensor 164, an electromyography (EMG) sensor 166, an oxygen sensor 168, an analyte sensor 174, a moisture sensor 176, a LiDAR sensor 178, or any combination thereof. Generally, each of the one or sensors 130 are configured to output sensor data that is received and stored in the memory device 114 or one or more other memory devices.

While the one or more sensors 130 are shown and described as including each of the pressure sensor 132, the flow rate sensor 134, the temperature sensor 136, the motion sensor 138, the microphone 140, the speaker 142, the RF receiver 146, the RF transmitter 148, the camera 150, the infrared sensor 152, the photoplethysmogram (PPG) sensor 154, the electrocardiogram (ECG) sensor 156, the electroencephalography (EEG) sensor 158, the capacitive sensor 160, the force sensor 162, the strain gauge sensor 164, the electromyography (EMG) sensor 166, the oxygen sensor 168, the analyte sensor 174, the moisture sensor 176, and the LiDAR sensor 178, more generally, the one or more sensors 130 can include any combination and any number of each of the sensors described and/or shown herein.

The one or more sensors 130 can be used to generate, for example, physiological data, audio data, image data, other data, or any combination thereof. As described in further detail herein, the sensor data from the one or more sensors 130 can be used to automatically determine adjustments for an oral appliance 122. In some cases, the sensor data can identify information about the user, the user's sleep session, the oral appliance 122, or another associated element.

In some cases, physiological data generated by one or more of the sensors 130 can be used by the control system 110 to determine a sleep-wake signal associated with a user during a sleep session and one or more sleep-related parameters. The sleep-wake signal can be indicative of one or more sleep states, including wakefulness, relaxed wakefulness, micro-awakenings, a rapid eye movement (REM) stage, a first non-REM stage (often referred to as "N1"), a second non-REM stage (often referred to as "N2"), a third non-REM stage (often referred to as "N3"), or any combination thereof. The sleep-wake signal can also be timestamped to indicate a time that the user enters the bed, a time that the user exits the bed, a time that the user attempts to fall asleep, etc. The sleep-wake signal can be measured by the sensor(s) 130 during the sleep session at a predetermined sampling rate, such as, for example, one sample per second, one sample per 30 seconds, one sample per minute, etc. Examples of the one or more sleep-related parameters that can be determined for the user during the sleep session based on the sleep-wake signal include a total time in bed, a total sleep time, a sleep onset latency, a wake-after-sleep-onset parameter, a sleep efficiency, a fragmentation index, or any combination thereof. Methods for determining sleep states and/or sleep stages from physiological data generated by one or more of the sensors, such as sensors 130, are described in, for example, WO 2014/047310, US 2014/0088373, WO 2017/132726, WO 2019/122413, and WO2019/122414

Physiological data and/or audio data generated by the one or more sensors 130 can also be used to determine a respiration signal associated with a user during a sleep session. The respiration signal is generally indicative of respiration or breathing of the user during the sleep session. The respiration signal can be indicative of, for example, a respiration rate, a respiration rate variability, an inspiration amplitude, an expiration amplitude, an inspiration-expiration ratio, a number of events per hour, a pattern of events, settings of the oral appliance 122, efficacy of the oral appliance 122 as currently set, or any combination thereof. The event(s) can include snoring, apneas, central apneas, obstructive apneas, mixed apneas, hypopneas, a restless leg, a sleeping disorder, choking, an increased heart rate, labored breathing, an asthma attack, an epileptic episode, a seizure, or any combination thereof. In some cases, this respiration signal can be used to facilitate determination of the adjustment for the oral appliance.

The pressure sensor 132 outputs pressure data (e.g., a pressure signal) that can be stored in the memory device 114 and/or analyzed by the processor 112 of the control system 110. In some implementations, the pressure sensor 132 is an air pressure sensor (e.g., barometric pressure sensor) that generates sensor data indicative of the respiration (e.g., inhaling and/or exhaling) of the user and/or ambient pressure. In such implementations, the pressure sensor 132 can be coupled to or integrated in the oral appliance 122 and/or oral appliance receptacle 126. The pressure sensor 132 can be, for example, a capacitive sensor, an electromagnetic sensor, a piezoelectric sensor, a strain-gauge sensor, an optical sensor, a potentiometric sensor, or any combination thereof. In one example, the pressure sensor 132 can be used to determine a blood pressure of a user.

The flow rate sensor 134 outputs flow rate data (e.g., a flow rate signal) that can be stored in the memory device 114 and/or analyzed by the processor 112 of the control system 110. In some implementations, the flow rate sensor 134 is used to determine an air flow rate at or adjacent the oral appliance 122. In such implementations, the flow rate sensor 134 can be coupled to or integrated in the oral appliance 122. The flow rate sensor 134 can be a mass flow rate sensor such as, for example, a rotary flow meter (e.g., Hall effect flow meters), a turbine flow meter, an orifice flow meter, an ultrasonic flow meter, a hot wire sensor, a vortex sensor, a membrane sensor, or any combination thereof. Examples of flow rate sensors (such as, for example, the flow rate sensor 134) are described in WO2012/012835.

The temperature sensor 136 outputs temperature data that can be stored in the memory device 114 and/or analyzed by the processor 112 of the control system 110. In some implementations, the temperature sensor 136 generates temperatures data indicative of a core body temperature of the user 210 (FIG. 2), a skin temperature of the user 210, a temperature inside the user's mouth via the oral appliance 122, a temperature in the oral appliance receptacle 126, an ambient temperature, or any combination thereof. The temperature sensor 136 can be, for example, a thermocouple sensor, a thermistor sensor, a silicon band gap temperature sensor or semiconductor-based sensor, a resistance temperature detector, or any combination thereof.

The microphone 140 outputs audio data that can be stored in the memory device 114 and/or analyzed by the processor 112 of the control system 110. The audio data generated by the microphone 140 is reproducible as one or more sound(s) during a sleep session (e.g., sounds from the user 210). The audio data form the microphone 140 can also be used to identify (e.g., using the control system 110) an event experienced by the user during the sleep session, as described in further detail herein. The microphone 140 can be coupled to or integrated in the oral appliance 122, the oral appliance receptacle 126, the user device 170, or the external device 171.

The speaker 142 outputs sound waves that are audible to a user of the system 100 (e.g., the user 210 of FIG. 2). The speaker 142 can be used, for example, as an alarm clock or to play an alert or message to the user 210 (e.g., in response to an event). In some implementations, the speaker 142 can be used to communicate the audio data generated by the microphone 140 to the user. The speaker 142 can be coupled to or integrated in the oral appliance 122, the oral appliance receptacle 126, the user device 170, or the external device 171.

The microphone 140 and the speaker 142 can be used as separate devices. In some implementations, the microphone 140 and the speaker 142 can be combined into an acoustic sensor 141 (e.g. a SONAR sensor), as described in, for example, WO 2018/050913 and WO2020/104465. In such implementations, the speaker 142 generates or emits sound waves at a predetermined interval and the microphone 140 detects the reflections of the emitted sound waves from the speaker 142. The sound waves generated or emitted by the speaker 142 have a frequency that is not audible to the human ear (e.g., below 20 Hz or above around 18 kHz) so as not to disturb the sleep of the user 210 or the bed partner 220 (FIG. 2). Based at least in part on the data from the microphone 140 and/or the speaker 142, the control system 110 can determine a location of the user 210 (FIG. 2) and/or one or more of the sleep-related parameters described in herein (e.g., an identified body position and/or a change in body position) and/or respiration-related parameters described in herein such as, for example, a respiration pattern, a respiration signal (from which, e.g., breath morphology may be determined), a respiration rate, an inspiration amplitude, an expiration amplitude, an inspiration-expiration ratio, a number of events per hour, a pattern of events, a sleep state, a sleep stage, or any combination thereof. In this context, a sonar sensor may be understood to concern an active acoustic sensing, such as by generating/transmitting ultrasound or low frequency ultrasound sensing signals (e.g., in a frequency range of about 17-23 kHz, 18-22 kHz, or 17-18 kHz, for example), through the air. Such a system may be considered in relation to WO2018/050913 and WO 2020/104465 mentioned above.

In some implementations, the sensors 130 include (i) a first microphone that is the same as, or similar to, the microphone 140, and is integrated in the acoustic sensor 141 and (ii) a second microphone that is the same as, or similar to, the microphone 140, but is separate and distinct from the first microphone that is integrated in the acoustic sensor 141.

The RF transmitter 148 generates and/or emits radio waves having a predetermined frequency and/or a predetermined amplitude (e.g., within a high frequency band, within a low frequency band, long wave signals, short wave signals, etc.). The RF receiver 146 detects the reflections of the radio waves emitted from the RF transmitter 148, and this data can be analyzed by the control system 110 to determine a location of the user 210 (FIG. 2) and/or one or more of the sleep-related parameters described herein. An RF receiver (either the RF receiver 146 and the RF transmitter 148 or another RF pair) can also be used for wireless communication between the control system 110, the oral appliance 122, the oral appliance receptacle 126, the one or more sensors 130, the user device 170, the external device 171, or any combination thereof. While the RF receiver 146 and RF transmitter 148 are shown as being separate and distinct elements in FIG. 1, in some implementations, the RF receiver 146 and RF transmitter 148 are combined as a part of an RF sensor 147. In some such implementations, the RF sensor 147 includes a control circuit. The specific format of the RF communication can be WiFi, Bluetooth, or the like.

In some implementations, the RF sensor 147 is a part of a mesh system. One example of a mesh system is a WiFi mesh system, which can include mesh nodes, mesh router(s), and mesh gateway(s), each of which can be mobile/movable or fixed. In such implementations, the WiFi mesh system includes a WiFi router and/or a WiFi controller and one or more satellites (e.g., access points), each of which include an RF sensor that the is the same as, or similar to, the RF sensor 147. The WiFi router and satellites continuously communicate with one another using WiFi signals. The WiFi mesh system can be used to generate motion data based on changes in the WiFi signals (e.g., differences in received signal strength) between the router and the satellite(s) due to an object or person moving partially obstructing the signals. The motion data can be indicative of motion, breathing, heart rate, gait, falls, behavior, etc., or any combination thereof.

The camera 150 outputs image data reproducible as one or more images (e.g., still images, video images, thermal images, or a combination thereof) that can be stored in the memory device 114. The image data from the camera 150 can be used by the control system 110 to determine one or more of the sleep-related parameters described herein. For example, the image data from the camera 150 can be used to identify a location of the user, to determine a time when the user 210 enters the bed 230 (FIG. 2), and to determine a time when the user 210 exits the bed 230. In some cases, image data from the camera 150 can be used by the control system 110 to detect or confirm occurrence of an event.

The infrared (IR) sensor 152 outputs infrared image data reproducible as one or more infrared images (e.g., still images, video images, or both) that can be stored in the memory device 114. The infrared data from the IR sensor 152 can be used to determine one or more sleep-related parameters during a sleep session, including a temperature of the user 210 and/or movement of the user 210. The IR sensor 152 can also be used in conjunction with the camera 150 when measuring the presence, location, and/or movement of the user 210. In some cases, infrared data from the IR sensor 152 can be used to detect or confirm occurrence of an event. The IR sensor 152 can detect infrared light having a wavelength between about 700 nm and about 1 mm, for example, while the camera 150 can detect visible light having a wavelength between about 380 nm and about 740 nm.

The PPG sensor 154 outputs physiological data associated with the user 210 (FIG. 2) that can be used to determine one or more sleep-related parameters, such as, for example, a heart rate, a heart rate variability, a cardiac cycle, respiration rate, an inspiration amplitude, an expiration amplitude, an inspiration-expiration ratio, estimated blood pressure parameter(s), or any combination thereof. The PPG sensor 154 can be worn by the user 210, embedded in clothing and/or fabric that is worn by the user 210, and/or embedded in and/or coupled to the oral appliance 122.

The ECG sensor 156 outputs physiological data associated with electrical activity of the heart of the user 210. In some implementations, the ECG sensor 156 includes one or more electrodes that are positioned on or around a portion of the user 210 during the sleep session. The physiological data from the ECG sensor 156 can be used, for example, to determine one or more of the sleep-related parameters described herein.

The EEG sensor 158 outputs physiological data associated with electrical activity of the brain of the user 210. In some implementations, the EEG sensor 158 includes one or more electrodes that are positioned on or around the scalp of the user 210 during the sleep session. The physiological data from the EEG sensor 158 can be used, for example, to determine a sleep state of the user 210 at any given time during the sleep session.

The capacitive sensor 160, the force sensor 162, and the strain gauge sensor 164 output data that can be stored in the memory device 114 and used by the control system 110 to determine one or more of the sleep-related parameters described herein. The EMG sensor 166 outputs physiological data associated with electrical activity produced by one or more muscles. The oxygen sensor 168 outputs oxygen data indicative of an oxygen concentration of gas (e.g., gas within the user's mouth as detected by a sensor in the oral appliance 122 or ambient gas as detected by a sensor in the oral appliance receptacle 126). The oxygen sensor 168 can be, for example, an ultrasonic oxygen sensor, an electrical oxygen sensor, a chemical oxygen sensor, an optical oxygen sensor, or any combination thereof. In some implementations, the one or more sensors 130 also include a galvanic skin response (GSR) sensor, a blood flow sensor, a respiration sensor, a pulse sensor, a sphygmomanometer sensor, an oximetry sensor, or any combination thereof.

The analyte sensor 174 can be used to detect the presence of an analyte in the breath of the user 210. The data output by the analyte sensor 174 can be stored in the memory device 114 and used by the control system 110 to determine the identity and concentration of any analytes in the breath of the user 210. In some implementations, the analyte sensor 174 is positioned within a mouth of the user 210 (e.g., coupled to and/or incorporated within the oral appliance 122) to detect analytes in breath being exhaled from the user 210's mouth. In still other implementations, the analyte sensor 174 can be positioned near the user 210's mouth, such as on an external device 171 located near the user's mouth. In some implementations, the analyte sensor 174 is a volatile organic compound (VOC) sensor that can be used to detect carbon-based chemicals or compounds. In some implementations, the analyte sensor 174 can also be used to detect whether the user 210 is breathing through their nose or mouth. For example, if the data output by an analyte sensor 174 positioned in or near the mouth of the user 210 detects the presence of an analyte, the control system 110 can use this data as an indication that the user 210 is breathing through their mouth.

The moisture sensor 176 outputs data that can be stored in the memory device 114 and used by the control system 110. The moisture sensor 176 can be used to detect moisture in various areas within or surrounding the user (e.g., at the oral appliance 122 or at the oral appliance receptacle 126). Thus, in some implementations, the moisture sensor 176 can be coupled to or integrated in the oral appliance 122 or the oral appliance receptacle 126.. In other implementations, the moisture sensor 176 is placed near any area where moisture levels need to be monitored. The moisture sensor 176 can also be used to monitor the humidity of the ambient environment surrounding the user 210, for example, the air inside the bedroom.

The Light Detection and Ranging (LiDAR) sensor 178 can be used for depth sensing. This type of optical sensor (e.g., laser sensor) can be used to detect objects and build three dimensional (3D) maps of the surroundings, such as of a living space. LiDAR can generally utilize a pulsed laser to make time of flight measurements. LiDAR is also referred to as 3D laser scanning. In an example of use of such a sensor, a fixed or mobile device (such as a smartphone) having a LiDAR sensor 166 can measure and map an area extending 5 meters or more away from the sensor. The LiDAR data can be fused with point cloud data estimated by an electromagnetic RADAR sensor, for example. The LiDAR sensor(s) 178 can also use artificial intelligence (AI) to automatically geofence RADAR systems by detecting and classifying features in a space that might cause issues for RADAR systems, such a glass windows (which can be highly reflective to RADAR). LiDAR can also be used to provide an estimate of the height of a person, as well as changes in height when the person sits down, or falls down, for example. LiDAR may be used to form a 3D mesh representation of an environment. In a further use, for solid surfaces through which radio waves pass (e.g., radio-translucent materials), the LiDAR may reflect off such surfaces, thus allowing a classification of different type of obstacles.

While shown separately in FIG. 1, any combination of the one or more sensors 130 can be integrated in and/or coupled to any one or more of the components of the system 100, including the oral appliance 122, the oral appliance receptacle 126, the control system 110, the user device 170, the external device 171, or any combination thereof. For example, the microphone 140 and speaker 142 is integrated in and/or coupled to the user device 170 and the temperature sensor 136 and/or motion sensor 138 are integrated in and/or coupled to the oral appliance 122. In some implementations, at least one of the one or more sensors 130 is not coupled to the oral appliance 122, the control system 110, the user device 170, or the external device 171, and is positioned generally adjacent to the user 210 during the sleep session (e.g., positioned on or in contact with a portion of the user 210, worn by the user 210, coupled to or positioned on the nightstand, coupled to the mattress, coupled to the ceiling, etc.). In some cases, one, some, or all of the one or more sensors 130 can be positioned external to the user. In some cases, one, some, or all of the one or more sensors 130 can be physically separated and not physically coupled to the oral appliance 122.

For example, as shown in FIG. 2, one or more of the sensors 130 can be located in a first position 250A on the nightstand 240 adjacent to the bed 230 and the user 210. Alternatively, one or more of the sensors 130 can be located in a second position on and/or in the mattress 232 (e.g., the sensor is coupled to and/or integrated in the mattress 232). Further, one or more of the sensors 130 can be located in a third position on the bed 230 (e.g., coupled to and/or integrated in a headboard, a footboard, or other location on the frame of the bed 230). One or more of the sensors 130 can also be located in a fourth position on a wall or ceiling that is generally adjacent to the bed 230 and/or the user 210. The one or more of the sensors 130 can also be located in a fifth position such that the one or more of the sensors 130 is coupled to and/or positioned on and/or inside a housing of the oral appliance 122 or oral appliance receptacle 126 of the oral appliance therapy system 120. Further, one or more of the sensors 130 can be located in a sixth position such that the sensor is coupled to and/or positioned on the user 210 (e.g., the sensor(s) is embedded in or coupled to fabric or clothing worn by the user 210 during the sleep session). More generally, the one or more of the sensors 130 can be positioned at any suitable location relative to the user 210 such that the sensor(s) 140 can generate sensor data (e.g., physiological data) associated with the user 210 and/or the bed partner 220 during one or more sleep session.

Referring back to FIG. 1, the user device 170 can include a processor (e.g., processor 112), a memory (e.g., memory 114), and a display device 172. The user device 170 can be, for example, a mobile device such as a smart phone, a tablet, a laptop, or the like. The display device 172 is generally used to display image(s) including still images, video images, or both. In some implementations, the display device 172 acts as a human-machine interface (HMI) that includes a graphic user interface (GUI) configured to display the image(s) and an input interface. The display device 172 can be an LED display, an OLED display, an LCD display, or the like. The input interface can be, for example, a touchscreen or touch-sensitive substrate, a mouse, a keyboard, or any sensor system configured to sense inputs made by a human user interacting with the user device 170.

In some cases, external device 171 can be a sleep-related device used to receive sensor data associated with a sleep session and/or control an action to affect the sleep session. As an example, the external device 171 can be a smart pillow, a smart mattress, smart bedding (e.g., sheets or blankets) or the like. In some cases, such an external device 171 can include one or more sensors 130 to receive sensor data associated with a sleep session. In some cases, such an external device 171 can be an actuatable or controllable device, which can be controlled to affect the sleep session. For example, such a device can include one or more inflatable bladders which can be controlled to inflate to urge the user into a particular sleeping positon or away from a particular sleeping position. For example, an inflatable bladder in a pillow or mattress can be used to urge a user from a supine-sleeping position towards a side-sleeping position. Other types of external devices 171 that are controllable (e.g., by control system 110) can be used to affect a user's sleep session in association with automatic adjustment of the oral appliance 122.

While the control system 110 and the memory device 114 are described and shown in FIG. 1 as being a separate and distinct component of the system 100, in some implementations, the control system 110 and/or the memory device 114 are integrated in the user device 170, the external device 171, the oral appliance receptacle 126, or any combination thereof. Alternatively, in some implementations, the control system 110 or a portion thereof (e.g., the processor 112) can be located in a cloud (e.g., integrated in a server, integrated in an Internet of Things (IoT) device, connected to the cloud, be subject to edge cloud processing, etc.), located in one or more servers (e.g., remote servers, local servers, etc., or any combination thereof.

While system 100 is shown as including all of the components described above, more or fewer components can be included in a system for automatically adjusting an oral appliance 122. For example, a first alternative system includes the control system 110, the oral appliance 122, and at least one of the one or more sensors 130. As another example, a second alternative system includes the oral appliance 122, an oral appliance receptacle 126, a plurality of the one or more sensors 130, and the user device 170. As yet another example, a third alternative system includes the oral appliance 122, at least one of the one or more sensors 130, and the user device 170. Thus, various systems for determining sleep-related parameters associated with a sleep session can be formed using any portion or portions of the components shown and described herein and/or in combination with one or more other components.

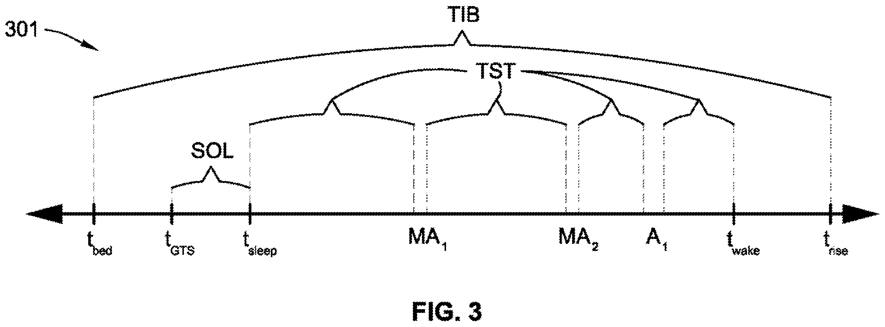

As used herein, a sleep session can be defined in a number of ways based on, for example, an initial start time and an end time. Referring to FIG. 3, an example timeline 300 for a sleep session is illustrated. The timeline 300 includes an enter bed time (t_{bed}), a go-to-sleep time (t_{GTS}), an initial sleep time (tₛₗₑₑₚ), a first micro-awakening MA₁ and a second micro-awakening MA₂, a wake-up time (t_{wake}), and a rising time (tᵣᵢₛₑ).

As used herein, a sleep session can be defined in multiple ways. For example, a sleep session can be defined by an initial start time and an end time. In some implementations, a sleep session is a duration where the user is asleep, that is, the sleep session has a start time and an end time, and during the sleep session, the user does not wake until the end time. That is, any period of the user being awake is not included in a sleep session. From this first definition of sleep session, if the user wakes ups and falls asleep multiple times in the same night, each of the sleep intervals separated by an awake interval is a sleep session.

Alternatively, in some implementations, a sleep session has a start time and an end time, and during the sleep session, the user can wake up, without the sleep session ending, so long as a continuous duration that the user is awake is below an awake duration threshold. The awake duration threshold can be defined as a percentage of a sleep session. The awake duration threshold can be, for example, about twenty percent of the sleep session, about fifteen percent of the sleep session duration, about ten percent of the sleep session duration, about five percent of the sleep session duration, about two percent of the sleep session duration, etc., or any other threshold percentage. In some implementations, the awake duration threshold is defined as a fixed amount of time, such as, for example, about one hour, about thirty minutes, about fifteen minutes, about ten minutes, about five minutes, about two minutes, etc., or any other amount of time.

**In** some implementations, a sleep session is defined as the entire time between the time in the evening at which the user first entered the bed, and the time the next morning when user last left the bed. Put another way, a sleep session can be defined as a period of time that begins on a first date (e.g., Monday, January 6, 2020) at a first time (e.g., 10:00 PM), that can be referred to as the current evening, when the user first enters a bed with the intention of going to sleep (e.g., not if the user intends to first watch television or play with a smart phone before going to sleep, etc.), and ends on a second date (e.g., Tuesday, January 7, 2020) at a second time (e.g., 7:00 AM), that can be referred to as the next morning, when the user first exits the bed with the intention of not going back to sleep that next morning.

Referring to FIG. 3, an exemplary timeline 300 for a sleep session is illustrated. The timeline 300 includes an enter bed time (t_{bed}), a go-to-sleep time (t_{GTS}), an initial sleep time (tₛₗₑₑₚ), a first micro-awakening MA₁, a second micro-awakening MA₂, an awakening A, a wake-up time (t_{wake}), and a rising time (tᵣᵢₛₑ).

The enter bed time t_{bed} is associated with the time that the user initially enters the bed (e.g., bed 230 in FIG. 2) prior to falling asleep (e.g., when the user lies down or sits in the bed). The enter bed time t_{bed} can be identified based on a bed threshold duration to distinguish between times when the user enters the bed for sleep and when the user enters the bed for other reasons (e.g., to watch TV). For example, the bed threshold duration can be at least about 10 minutes, at least about 20 minutes, at least about 30 minutes, at least about 45 minutes, at least about 1 hour, at least about 2 hours, etc. While the enter bed time t_{bed} is described herein in reference to a bed, more generally, the enter time t_{bed} can refer to the time the user initially enters any location for sleeping (e.g., a couch, a chair, a sleeping bag, etc.).

The go-to-sleep time (GTS) is associated with the time that the user initially attempts to fall asleep after entering the bed (t_{bed}). For example, after entering the bed, the user may engage in one or more activities to wind down prior to trying to sleep (e.g., reading, watching TV, listening to music, using the user device 170, etc.). The initial sleep time (tₛₗₑₑₚ) is the time that the user initially falls asleep. For example, the initial sleep time (tₛₗₑₑₚ) can be the time that the user initially enters the first non-REM sleep stage.

The wake-up time t_{wake} is the time associated with the time when the user wakes up without going back to sleep (e.g., as opposed to the user waking up in the middle of the night and going back to sleep). The user may experience one of more unconscious microawakenings (e.g., microawakenings MA₁ and MA₂) having a short duration (e.g., 5 seconds, 10 seconds, 30 seconds, 1 minute, etc.) after initially falling asleep. In contrast to the wake-up time t_{wake}, the user goes back to sleep after each of the microawakenings MA₁ and MA₂. Similarly, the user may have one or more conscious awakenings (e.g., awakening A) after initially falling asleep (e.g., getting up to go to the bathroom, attending to children or pets, sleep walking, etc.). However, the user goes back to sleep after the awakening A. Thus, the wake-up time t_{wake} can be defined, for example, based on a wake threshold duration (e.g., the user is awake for at least 15 minutes, at least 20 minutes, at least 30 minutes, at least 1 hour, etc.).

Similarly, the rising time tᵣᵢₛₑ is associated with the time when the user exits the bed and stays out of the bed with the intent to end the sleep session (e.g., as opposed to the user getting up during the night to go to the bathroom, to attend to children or pets, sleep walking, etc.). In other words, the rising time tᵣᵢₛₑ is the time when the user last leaves the bed without returning to the bed until a next sleep session (e.g., the following evening). Thus, the rising time tᵣᵢₛₑ can be defined, for example, based on a rise threshold duration (e.g., the user has left the bed for at least 15 minutes, at least 20 minutes, at least 30 minutes, at least 1 hour, etc.). The enter bed time t_{bed} time for a second, subsequent sleep session can also be defined based on a rise threshold duration (e.g., the user has left the bed for at least 4 hours, at least 6 hours, at least 8 hours, at least 12 hours, etc.).

As described above, the user may wake up and get out of bed one more times during the night between the initial t_{bed} and the final tᵣᵢₛₑ. In some implementations, the final wake-up time t_{wake} and/or the final rising time tᵣᵢₛₑ that are identified or determined based on a predetermined threshold duration of time subsequent to an event (e.g., falling asleep or leaving the bed). Such a threshold duration can be customized for the user. For a standard user which goes to bed in the evening, then wakes up and goes out of bed in the morning any period (between the user waking up (t_{wake}) or raising up (tᵣᵢₛₑ), and the user either going to bed (t_{bed}), going to sleep (T_{GTS}) or falling asleep (tₛₗₑₑₚ) of between about 12 and about 18 hours can be used. For users that spend longer periods of time in bed, shorter threshold periods may be used (e.g., between about 8 hours and about 14 hours). The threshold period may be initially selected and/or later adjusted based on the system monitoring the user's sleep behavior.

The total time in bed (TIB) is the duration of time between the time enter bed time t_{bed} and the rising time tᵣᵢₛₑ. The total sleep time (TST) is associated with the duration between the initial sleep time and the wake-up time, excluding any conscious or unconscious awakenings and/or micro-awakenings therebetween. Generally, the total sleep time (TST) will be shorter than the total time in bed (TIB) (e.g., one minute short, ten minutes shorter, one hour shorter, etc.). For example, referring to the timeline 300 of FIG. 3, the total sleep time (TST) spans between the initial sleep time tₛₗₑₑₚ and the wake-up time t_{wake}, but excludes the duration of the first micro-awakening MA₁, the second micro-awakening MA₂, and the awakening A. As shown, in this example, the total sleep time (TST) is shorter than the total time in bed (TIB).

In some implementations, the total sleep time (TST) can be defined as a persistent total sleep time (PTST). In such implementations, the persistent total sleep time excludes a predetermined initial portion or period of the first non-REM stage (e.g., light sleep stage). For example, the predetermined initial portion can be between about 30 seconds and about 20 minutes, between about 1 minute and about 10 minutes, between about 3 minutes and about 5 minutes, etc. The persistent total sleep time is a measure of sustained sleep, and smooths the sleep-wake hypnogram. For example, when the user is initially falling asleep, the user may be in the first non-REM stage for a very short time (e.g., about 30 seconds), then back into the wakefulness stage for a short period (e.g., one minute), and then goes back to the first non-REM stage. In this example, the persistent total sleep time excludes the first instance (e.g., about 30 seconds) of the first non-REM stage.

In some implementations, the sleep session is defined as starting at the enter bed time (t_{bed}) and ending at the rising time (tᵣᵢₛₑ), i.e., the sleep session is defined as the total time in bed (TIB). In some implementations, a sleep session is defined as starting at the initial sleep time (tₛₗₑₑₚ) and ending at the wake-up time (t_{wake}). In some implementations, the sleep session is defined as the total sleep time (TST). In some implementations, a sleep session is defined as starting at the go-to-sleep time (T_{GTS}) and ending at the wake-up time (t_{wake}). In some implementations, a sleep session is defined as starting at the go-to-sleep time (T_{GTS}) and ending at the rising time (tᵣᵢₛₑ). In some implementations, a sleep session is defined as starting at the enter bed time (t_{bed}) and ending at the wake-up time (t_{wake}). In some implementations, a sleep session is defined as starting at the initial sleep time (tₛₗₑₑₚ) and ending at the rising time (tᵣᵢₛₑ).

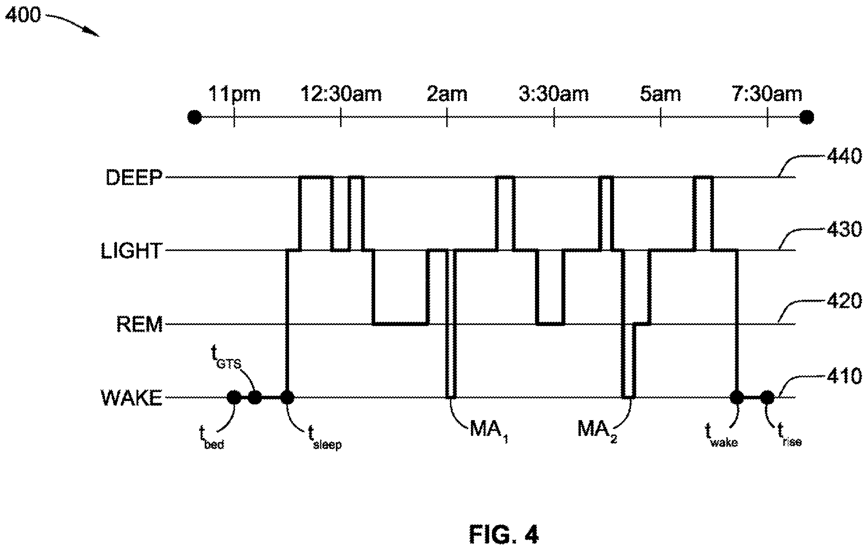

Referring to FIG. 4, an exemplary hypnogram 400 corresponding to the timeline 300 (FIG. 3), according to some implementations, is illustrated. As shown, the hypnogram 400 includes a sleep-wake signal 401, a wakefulness stage axis 410, a REM stage axis 420, a light sleep stage axis 430, and a deep sleep stage axis 440. The intersection between the sleep-wake signal 401 and one of the axes 410-440 is indicative of the sleep stage at any given time during the sleep session.

The sleep-wake signal 401 can be generated based on physiological data associated with the user (e.g., generated by one or more of the sensors 130 described herein). The sleep-wake signal can be indicative of one or more sleep states, including wakefulness, relaxed wakefulness, microawakenings, a REM stage, a first non-REM stage, a second non-REM stage, a third non-REM stage, or any combination thereof. In some implementations, one or more of the first non-REM stage, the second non-REM stage, and the third non-REM stage can be grouped together and categorized as a light sleep stage or a deep sleep stage. For example, the light sleep stage can include the first non-REM stage and the deep sleep stage can include the second non-REM stage and the third non-REM stage. While the hypnogram 400 is shown in FIG. 4 as including the light sleep stage axis 430 and the deep sleep stage axis 440, in some implementations, the hypnogram 400 can include an axis for each of the first non-REM stage, the second non-REM stage, and the third non-REM stage. In other implementations, the sleep-wake signal can also be indicative of a respiration signal, a respiration rate, an inspiration amplitude, an expiration amplitude, an inspiration-expiration ratio, a number of events per hour, a pattern of events, or any combination thereof. Information describing the sleep-wake signal can be stored in the memory device 114.

The hypnogram 400 can be used to determine one or more sleep-related parameters, such as, for example, a sleep onset latency (SOL), wake-after-sleep onset (WASO), a sleep efficiency (SE), a sleep fragmentation index, sleep blocks, or any combination thereof.

The sleep onset latency (SOL) is defined as the time between the go-to-sleep time (t_{GTS}) and the initial sleep time (tₛₗₑₑₚ). In other words, the sleep onset latency is indicative of the time that it took the user to actually fall asleep after initially attempting to fall asleep. In some implementations, the sleep onset latency is defined as a persistent sleep onset latency (PSOL). The persistent sleep onset latency differs from the sleep onset latency in that the persistent sleep onset latency is defined as the duration time between the go-to-sleep time and a predetermined amount of sustained sleep. In some implementations, the predetermined amount of sustained sleep can include, for example, at least 10 minutes of sleep within the second non-REM stage, the third non-REM stage, and/or the REM stage with no more than 2 minutes of wakefulness, the first non-REM stage, and/or movement therebetween. In other words, the persistent sleep onset latency requires up to, for example, 8 minutes of sustained sleep within the second non-REM stage, the third non-REM stage, and/or the REM stage. In other implementations, the predetermined amount of sustained sleep can include at least 10 minutes of sleep within the first non-REM stage, the second non-REM stage, the third non-REM stage, and/or the REM stage subsequent to the initial sleep time. In such implementations, the predetermined amount of sustained sleep can exclude any micro-awakenings (e.g., a ten second micro-awakening does not restart the 10-minute period).

The wake-after-sleep onset (WASO) is associated with the total duration of time that the user is awake between the initial sleep time and the wake-up time. Thus, the wake-after-sleep onset includes short and micro-awakenings during the sleep session (e.g., the micro-awakenings MA₁ and MA₂ shown in FIG. 4), whether conscious or unconscious. In some implementations, the wake-after-sleep onset (WASO) is defined as a persistent wake-after-sleep onset (PWASO) that only includes the total durations of awakenings having a predetermined length (e.g., greater than 10 seconds, greater than 30 seconds, greater than 60 seconds, greater than about 5 minutes, greater than about 10 minutes, etc.)

The sleep efficiency (SE) is determined as a ratio of the total time in bed (TIB) and the total sleep time (TST). For example, if the total time in bed is 8 hours and the total sleep time is 7.5 hours, the sleep efficiency for that sleep session is 93.75%. The sleep efficiency is indicative of the sleep hygiene of the user. For example, if the user enters the bed and spends time engaged in other activities (e.g., watching TV) before sleep, the sleep efficiency will be reduced (e.g., the user is penalized). In some implementations, the sleep efficiency (SE) can be calculated based on the total time in bed (TIB) and the total time that the user is attempting to sleep. In such implementations, the total time that the user is attempting to sleep is defined as the duration between the go-to-sleep (GTS) time and the rising time described herein. For example, if the total sleep time is 8 hours (e.g., between 11 PM and 7 AM), the go-to-sleep time is 10:45 PM, and the rising time is 7:15 AM, in such implementations, the sleep efficiency parameter is calculated as about 94%.

The fragmentation index is determined based at least in part on the number of awakenings during the sleep session. For example, if the user had two micro-awakenings (e.g., micro-awakening MA₁ and micro-awakening MA₂ shown in FIG. 4), the fragmentation index can be expressed as 2. In some implementations, the fragmentation index is scaled between a predetermined range of integers (e.g., between 0 and 10).

The sleep blocks are associated with a transition between any stage of sleep (e.g., the first non-REM stage, the second non-REM stage, the third non-REM stage, and/or the REM) and the wakefulness stage. The sleep blocks can be calculated at a resolution of, for example, 30 seconds.

In some implementations, the systems and methods described herein can include generating or analyzing a hypnogram including a sleep-wake signal to determine or identify the enter bed time (t_{bed}), the go-to-sleep time (t_{GTS}), the initial sleep time (tₛₗₑₑₚ), one or more first micro-awakenings (e.g., MA₁ and MA₂), the wake-up time (t_{wake}), the rising time (tᵣᵢₛₑ), or any combination thereof based at least in part on the sleep-wake signal of a hypnogram.

In other implementations, one or more of the sensors 130 can be used to determine or identify the enter bed time (t_{bed}), the go-to-sleep time (t_{GTS}), the initial sleep time (tₛₗₑₑₚ), one or more first micro-awakenings (e.g., MA₁ and MA₂), the wake-up time (t_{wake}), the rising time (tᵣᵢₛₑ), or any combination thereof, which in turn define the sleep session. For example, the enter bed time t_{bed} can be determined based on, for example, data generated by the motion sensor 138, the microphone 140, the camera 150, or any combination thereof. The go-to-sleep time can be determined based on, for example, data from the motion sensor 138 (e.g., data indicative of no movement by the user), data from the camera 150 (e.g., data indicative of no movement by the user and/or that the user has turned off the lights) data from the microphone 140 (e.g., data indicative of the using turning off a TV), data from the user device 170 (e.g., data indicative of the user no longer using the user device 170), data from a sensor in the oral appliance 122 (e.g., data indicating insertion of the oral appliance 122), data from a sensor in the oral appliance receptacle 126 (e.g., data indicating removal of the oral appliance 122 from the oral appliance receptacle 126), data from a sensor in the external device 171, or any combination thereof.

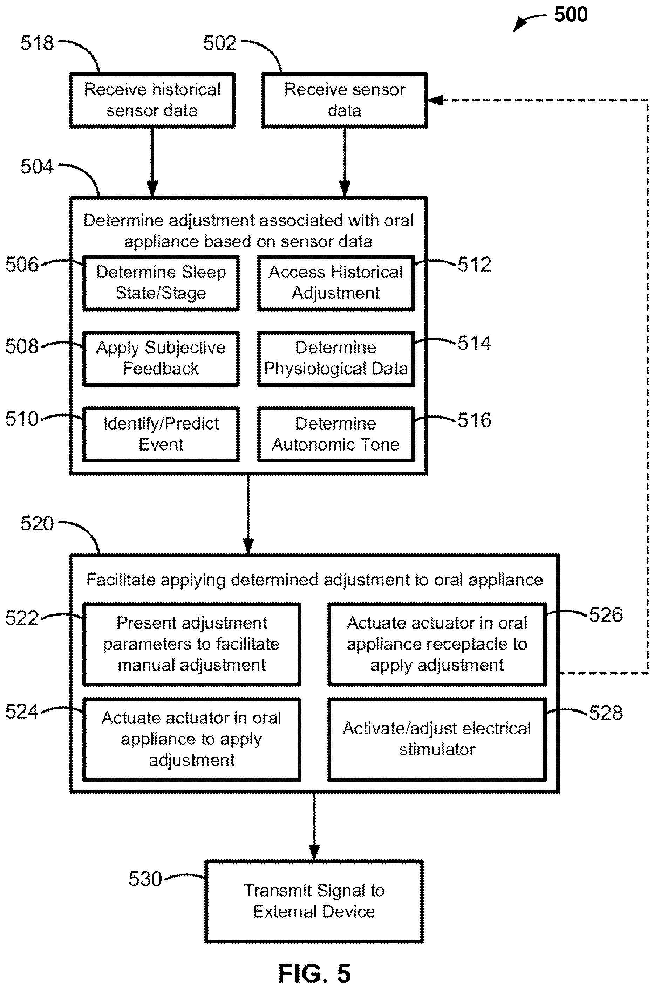

FIG. 5 is a flowchart depicting a process 500 for automatically adjusting an oral appliance, according to certain aspects of the present disclosure. Process 500 can be carried out by system 100 of FIG. 1, such as by control system 110 and other components of a system 100. Process 500 can be used to provide adjustments to an oral appliance, an oral appliance receptacle, and optionally an external device, such as oral appliance 122, oral appliance receptacle 126, and external device 171 of FIG. 1, respectively. Process 500 can be used to automatically adjust any oral appliance, such as a mandibular repositioning device.

At block 502, sensor data can be received. Sensor data can be received from one or more sensors, such as one or more sensors 130 of FIG. 1. Sensor data can be received from one or more sensors external to a user, although that need not always be the case. The sensor data can include physiological data, audio data, video data, movement data, environmental data, or other data associated with a user of an oral appliance (e.g., a user wearing the oral appliance in their mouth before, during, or after sleeping, and/or the user not wearing the oral appliance in their mouth during these times).

Receiving sensor data at block 502 can occur continuously over time (e.g., as sensor data is collected by the one or more sensors) or in bulk (e.g., as a collection of data representing sensor data from a period of time, such as an entire sleep session). Receiving sensor data at block 502 can occur in real-time (e.g., receiving sensor data during a sleep session as the one or more sensors collect the data associated with the sleep session), or asynchronously (e.g., receiving sensor data associated with a period of time in the past, such as data associated with a previous night's sleep session or sensor data delayed by a duration of time).

In some cases, optional block 518 can include receiving historical sensor data. Historical sensor data can include any sensor data that was collected prior to collection of the sensor data from block 502. In some cases, the historical sensor data at block 518 can include sensor data that was received in a previous occurrence of block 502, such as sensor data from earlier in a sleep session or sensor data from a previous sleep session. In some cases, historical sensor data received at block 518 can be compared with sensor data received at block 502 to determine an efficacy of previous oral appliance therapy (e.g., the efficacy of previous adjustments to the oral appliance).

At block 504, an adjustment associated with the oral appliance can be determined based on the sensor data. The system can make use of the sensor data received at block 502 to determine one or more adjustments associated with the oral appliance (e.g., one or more adjustments of one or more adjustable aspects of the oral appliance).

The adjustment determined at block 504 can be intended to achieve a desired purpose, such as to treat a sleep condition of the user, to improve a quality of sleep of the user (e.g., increase TST, decrease SOL, improve time spent in different stages of wakefulness (e.g., reduced fatigue, improved alertness, or the like)), improve comfort and/or efficacy of the oral appliance, or other purpose. In some cases, the desired purpose of the determined adjustment at block 504 can be dependent upon the sensor data 502, an internal clock, and/or other data. For example, in some cases, the purpose of the determined adjustment may be to improve comfort before onset of sleep, after which the determined adjustment may be to improve efficacy of the oral appliance. Such an example can also be considered to improve the quality of sleep of the user by reducing SOL by promoting comfort before onset of sleep and by increasing TST by promoting treatment efficacy of the oral appliance after onset of sleep.

In some cases, determining the adjustment at block 504 can include determining a sleep state and/or a sleep stage at block 506. The sleep state and/or sleep stage can be determined based on the received sensor data from block 502. Depending on the sleep state and/or sleep stage, the system can make different adjustments. As an example, the process can put the oral appliance into a comfort-prioritized state when it is determined that the user is awake or in light sleep (e.g., N1 sleep), and the process can put the oral appliance into a therapy-prioritized state when it is determined that the user is in deep sleep, optionally specifically in REM sleep. In some cases, the adjustments to be made to put the oral appliance in the desired state can be tuned to prior knowledge or detection of a user's non-REM vs. REM likelihood. In some cases, adjustments may be made to the oral appliance throughout sleep cycles, relaxing when the user is predicted to move from a slow wave sleep (SWS) or REM to a light stage of sleep.

In an example, if it is determined that the user is in a pre-sleep state (e.g., a time before tₛₗₑₑₚ), the system may use a preset pre-sleep adjustment. The preset pre-sleep adjustment can be a preset adjustment or preset algorithm for determining an adjustment. The pre-sleep adjustment can be designed to improve comfort of the user as the user is trying to fall asleep. Ideally, the pre-sleep adjustment may reduce SOL. The pre-sleep adjustment can put the oral appliance into a comfort-prioritized state.

As another example, if it is determined that the user is in a post-onset-of-sleep state (e.g., a time after t*ₛₗₑₑₚ*)*,* the system may use a preset post-onset-of-sleep adjustment. The preset post-onset-of-sleep adjustment can be a preset adjustment or preset algorithm for determining an adjustment. The post-onset-of-sleep adjustment can be designed to improve treatment efficacy of the oral appliance while the user is asleep. Ideally, the post-onset-of-sleep adjustment may avoid, reduce, or minimize the occurrence of events, such as apnea events. The post-onset-of-sleep adjustment can put the oral appliance into a treatment-prioritized state.

In some cases, the system can use a determined sleep state and/or sleep stage to deny, permit, pause, or resume adjustments that are desired to be implemented. For example, if the system is attempting to adjust the oral appliance over a period of time (e.g., tens or hundreds of seconds), the system may pause any adjustment to the oral appliance if it is determined that the user is in a micro-awakening state, the resume adjustment after the micro-awakening state has ended. Such control of adjustments can reduce the risk of accidentally awakening the user or otherwise negatively affecting the user's sleep session.

In some cases, determining the adjustment at block 504 can include applying subjective feedback at block 508. Subjective feedback can include any subjective feedback received by the user or another individual, such as a medical professional or a caregiver. The subjective feedback can be volunteered or requested. In some cases, subjective feedback is associated with a historical sleep session, with or without oral appliance therapy in use. In an example, the user may provide feedback as a response to a prompt, such as a positive response to a question about whether or not the user feels well-rested after a sleep session. In that example, the system may determine an adjustment based on that positive response.

In some cases, subjective feedback is associated with the oral appliance, with or without being associated with a sleep session. In an example, the user may provide feedback as a response to a prompt, such as a negative response to a question about whether or not the oral appliance feels comfortable in the user's mouth. In that example, the system may determine an adjustment based on that negative response. In another example, the user may volunteer feedback by interacting with a control on a user device (e.g., a smartphone) or on an oral appliance receptacle. Based on the selected control, the system may determine an adjustment. For example, if the user selects a control associated with "too tight" or "too loose," the system can determine an adjustment that would make the oral appliance feel more loose or more tight, respectively.

**In** some cases, similar controls can be used generally at block 504 to directly control adjustment of the oral appliance. For example, a control associated with "lengthen strut" can be used to force the system to determine an adjustment at block 504 that would result in lengthening of the associated strut in the oral appliance.

**In** some cases, determining the adjustment at block 504 can include identifying and/or predicting an event at block 510. Identifying an event at block 510 can include using the received sensor data from block 502 to identify that an event has occurred, and optionally classify the event or determine other information associated with the event. Upon identification of the occurrence of an event, the system can make a desired adjustment, such as an adjustment designed to stop, reduce, or minimize the event or effects of the event. In an example, upon determining that an apnea event has occurred, the system can use a preset post-apnea adjustment. The preset post-apnea adjustment can be a preset adjustment or preset algorithm for determining an adjustment. The post-apnea adjustment can be designed to improve treatment efficacy of the oral appliance in a manner that is specifically effective or desirable following an apnea event. For example, the post-apnea adjustment may put the oral appliance into a post-event state (e.g., a post-apnea-event state). In the post-event state, the oral appliance may be especially effective at stopping, reducing, or minimizing the event or the occurrence of a subsequent event. However, since the oral appliance may not be as comfortable in the post-event state, the system may make further adjustments to revert the oral appliance to a more comfortable state (e.g., a previous state, such as a post-onset-of-sleep state) after the event has passed (e.g., after the event is no longer detected or after a preset period of no longer detecting an event).

Predicting an event at block 510 can include using the received sensor data from block 502 to determine that a future event is likely to occur, and optionally classify the future event or determine other information associated with the future event. Predicting the future event can include determining that the likelihood of the future event occurring is above a threshold amount. Predicting the future event can be based on historical physiological data, historical sleep state or sleep stage data, historical adjustments or settings of the oral appliance, or any combination thereof, and which can be compared to current physiological data, current sleep state or sleep stage data, adjustments or settings of the oral appliance, or any combination thereof, to predict the future event. Upon prediction of the future event, the system can make a desired adjustment, such as an adjustment designed to avoid, stop, reduce, or minimize the future event or effects of the future event. In an example, upon determining that a future apnea event is likely to occur, the system can use a preset pre-apnea adjustment. The preset pre-apnea adjustment can be a preset adjustment or preset algorithm for determining an adjustment. The pre-apnea adjustment can be designed to improve treatment efficacy of the oral appliance in a manner that is specifically effective or desirable to avoid, reduce, or minimize a future apnea event. For example, the pre-apnea adjustment may put the oral appliance into a pre-event state (e.g., a pre-apnea-event state). In the pre-event state, the oral appliance may be especially effective at avoiding, reducing, or minimizing the future event. However, since the oral appliance may not be as comfortable in the pre-event state, the system may make further adjustments to revert the oral appliance to a previous state (e.g., a post-onset-of-sleep state) after the future event has passed (e.g., after the future event is no longer detected or after a preset period of no longer detecting the future event) or after a certain duration of time has passed and no event has occurred (e.g., a duration of time following prediction of the future event or a duration of time ending at or after the time when the future event is predicted to occur).

In some cases, determining the adjustment at block 504 can include accessing one or more historical adjustments (e.g., historical adjustment data) at block 512. The one or more historical adjustments can be used to help determine the current adjustment to be made, such as the type and/or extent of the adjustment to be made. In an example, historical adjustment data can be compared to sensor data (e.g., received sensor data at block 502 and/or historical sensor data received at block 518) to determine the effect of a previous adjustment (e.g., an immediately previous adjustment or other historical adjustment. If the previous adjustment was not as effective as desired or was overly uncomfortable, the system can adapt and determine an adjustment that is suitable for the desired outcome. For example, if a previous adjustment failed to decrease, or increased, the frequency of apnea events, the current adjustment can include reverting some or all of the previous adjustment.

In some cases, determining the adjustment at block 504 can include determining, at block 514, physiological data based on the sensor data from block 502. The physiological data can be used to determine the desired adjustment. Determining physiological data can include determining a breathing rate, a heart rate, a blood oxygenation level, a number of apnea events, a frequency of apnea events, or other physiological data associated with the user. The physiological data can be associated with the user during a sleep session or outside of a sleep session. For example, a certain adjustment may be especially useful when it is determined that the user's blood oxygenation level drops below a threshold while the user is asleep. As another example, a certain adjustment may be especially useful to improve the user's sleep session when it is determined that the user's average breathing rate or heart rate for a period of time prior to the sleep session exceeds a defined threshold. In some cases, determining physiological data at block 514 can include determining that an apnea event that has occurred is an obstructive apnea event or a central apnea event. If it is determined that a central apnea event has occurred, an indication can be presented to the user or another (e.g., a medical professional or caregiver) indicating that a central apnea event was detected, and optionally indicating that the oral appliance may be unsuitable for treating central apnea.

In some cases, determining an adjustment using the determined physiological data at block 514 can include identifying a desired change in the physiological data (e.g., a desire to increase blood oxygenation level), then determining an adjustment that is expected to effect that desired change in physiological data. Because of the ability for process 500 to operate as a feedback loop, as described in further detail herein, the system can monitor the physiological data over time to determine whether or not the determined adjustments are successful in effecting the desired change in physiological data. The adjustments made and resultant changes in physiological data can be used to train a model (e.g., a machine learning model) associated with an individual sleep session, an individual user, an individual oral appliance, and/or an individual style of oral appliance. Thus, as the system is used over time, the system can more accurately and effectively determine adjustments to be made.

In some cases, determining the adjustment at block 504 can include determining, at block 516, an autonomic tone based on sensor data from block 502. The autonomic tone can be used to determine the desired adjustment. For example, if a change in autonomic tone is detected that is indicative of increased sympathetic nerve activity (e.g., a change in autonomic tone associated with a fight-or-flight response), the system may use an adjustment that reverts a previous adjustment, slows the rate of a current adjustment, or otherwise moves the oral appliance into a more comfortable state. Thus, determination of autonomic tone at block 516 can help avoid inadvertently awakening the user.

In some cases, determining the adjustment at block 504 can include any combination of one or more of block 506, block 508, block 510, block 512, block 514, and block 516. In some cases, determining the adjustment at block 504 can include other blocks in addition to or alternative to any of block 506, block 508, block 510, block 512, block 514, and block 516.

At block 520, the system can facilitate applying the determined adjustment to the oral appliance. In some cases, facilitating applying the determined adjustment includes taking a direct action to apply the determined adjustment to the oral appliance. For example, such a direct action can include sending a signal to the oral appliance to cause the oral appliance to make the adjustment or sending a signal to the oral appliance receptacle to have the oral appliance receptacle adjust the oral appliance. In some cases, however, facilitating applying the determined adjustment can include taking an action that indirectly results in the determined adjustment being applied to the oral appliance. For example, such an indirect action can include taking an action that can help the user, another individual (e.g., a medical professional or a caregiver), or another system apply the determined adjustment.

In some cases, facilitating applying the determined adjustment at block 520 can include transmitting a signal (e.g., an adjustment signal) to the oral appliance. The signal can be transmitted to the oral appliance through any suitable technique, such as wired or wireless transmission. Generally, wireless transmission may be preferred. The signal can be transmitted to the oral appliance while the oral appliance is being used by the user (e.g., is worn in the mouth of the user), although that need not always be the case. In some cases, a signal can be transmitted to the oral appliance while the oral appliance is being stored in an oral appliance storage receptacle.

In some cases, facilitating applying the determined adjustment at block 520 can include presenting one or more adjustment parameters to facilitate manual adjustment at block 522. An adjustment parameter can include an indication of what adjustment must be made (e.g., "Shorten the coupling strut by 1 mm") and/or directions (e.g., step-by-step directions) of how to effect the desired adjustment (e.g., "Remove Part A from Slot A then insert Part A into Slot B"). Presenting an adjustment parameter can include displaying the adjustment parameter on a device, such as on an user device (e.g., a smartphone or a computer), on a display of an oral appliance receptacle, and/or on the oral appliance (e.g., lighting a region of the oral appliance where the adjustment is to occur). In some cases, presenting an adjustment parameter can include displaying the adjustment parameter as an overlay on a graphic of the oral appliance and/or as an augmented reality overlay on an image of the oral appliance (e.g., a live image of the oral appliance or a non-live image of the oral appliance). In some cases, presenting an adjustment parameter can include generating a printout of the adjustment parameter. As a result of presenting the adjustment parameter at block 522, the user or another individual can be prompted and instructed to manually adjust the oral appliance, and the oral appliance can be manually adjusted. As used herein, the term "automatic adjustment" with respect to an oral appliance can include automatically presenting adjustment parameters to facilitate manual adjustment of an oral appliance.

In some cases, facilitating applying the determined adjustment at block 520 can include actuating an actuator in the oral appliance to apply the adjustment at block 524. At block 524, the system can cause a signal to be transmitted to the oral appliance to cause one or more actuators of the oral appliance to actuate. In cases where block 524 is taking place while the user is current using the oral appliance, the technique for transmitting the signal is generally wireless transmission. However, in some cases, block 524 can occur after the oral appliance is removed from the user's oral cavity, such as when the oral appliance is placed in an oral appliance receptacle. In such cases, actuators in the oral appliance might be actuated to effect the adjustment through a wired connection using a set of exposed contacts on the oral appliance. Generally, however, wireless transmission may be preferred. In response to the transmitted signal, the one or more actuators of the oral appliance can be actuated, which can manipulate the one or more adjustable aspects of the oral appliance, thus effecting the determined adjustment.

In some cases, facilitating applying the determined adjustment at block 520 can include actuating an actuator in an oral appliance receptacle to apply the adjustment at block 526. At block 526, the system can cause the oral appliance receptacle to actuate one or more actuators within the oral appliance receptacle to implement the determined adjustment on the oral appliance. In some cases, block 526 includes waiting for a start command from a user and/or waiting for an indication that the oral appliance is received by the oral appliance receptacle (e.g., receiving sensor data indicative that the oral appliance is received by the oral appliance receptacle). In some cases, block 526 includes automatically aligning the oral appliance within the receiving space of the oral appliance receptacle, such as using sensor data. In some cases, block 526 can include using sensor data to determine if the oral appliance is properly seated within the receiving space of the oral appliance receptacle, only proceeding to make the adjustment if the oral appliance is properly seated, and optionally warning the user if the oral appliance is not properly seated. In some cases, block 526 can include using sensor data to determine whether the adjustment was successful.

In some cases, facilitating applying the determined adjustment at block 520 can include activating and/or adjusting an electrical stimulator in the oral appliance at block 528. In some cases, an oral appliance can include an optional electrical stimulator. The electrical stimulator can include a source of voltage and electrodes for conveying the voltage to tissue of the user. The electrical stimulator can be designed to stimulate muscle and/or tissues in the user's oral cavity, such as the tongue (e.g., via a tongue muscle or tongue nerves). The electrical stimulator can be used to apply additional treatment to a sleep condition, such as contraction of the tongue to stop, avoid, reduce, or minimize an apnea event. Activating an electrical stimulator at block 528 can include transmitting a signal that is received by the oral appliance and that causes the oral appliance to initiate an electrical stimulation. Adjusting the electrical stimulator at block 528 can include adjusting a setting (e.g., a software setting) of the oral appliance associated with the electrical stimulator. A setting associated with the electrical stimulator can control any suitable aspect of the electrical stimulator, including how and when the electrical stimulus is generated and/or delivered to the user.

In some cases, facilitating applying the determined adjustment at block 520 can include any combination of one or more of block 522, block 524, block 526, and block 528. In some cases, facilitating applying the determined adjustment at block 520 can include other actions in addition to or alternative to any of block 522, block 524, block 526, and block 528. For example, in some cases, facilitating applying the determined adjustment at block 520 can include adjusting a software setting of the oral appliance. Such a software setting can be adjusted similar to adjusting the electrical stimulator at block 528, but may not relate to use of an electrical stimulator.

In some cases, after an adjustment has been facilitated at block 520, such as after an adjustment has been effected, process 500 can continue again at a new instance of block 502 to receive additional sensor data and a new instance of block 504 to determine one or more additional adjustments. Based on the one or more additional adjustments, a new instance of block 520 can allow the system to facilitate applying the determined additional adjustment to the oral appliance. In this fashion, the system can dynamically adjust the oral appliance in a feedback loop. Thus, the system can continuously and repeatedly use feedback from one or more previous adjustments (e.g., an immediately previous adjustment) to determine and implement a new adjustment.

In some optional cases, in addition to applying the determined adjustment at block 520, process 500 can include transmitting a signal to an external device at block 530. The signal transmitted at block 530 can adjust a setting of the external device, such as to cause the external device to take an action that affects the user's sleep session, such as an action that affects the efficacy of the oral appliance therapy. In some cases, the signal transmitted at block 530 only occurs after it is determined (e.g., from analysis of the received sensor data from block 502) that the user is using the oral appliance. In some cases, the signal transmitted to the external device causes the external device to urge the user into a desired sleeping position. In some cases, transmitting the signal to the external deice at block 530 only occurs if other conditions are met, such as if it is determined (e.g., based on sensor data) that the user is sleeping in an undesired sleeping position or if the user is in a particular sleep state or sleep stage.

In an example, when the received sensor data at block 502 is indicative that the user is using an oral appliance, and optionally indicative that the user is sleeping in a supine-sleeping position, the system can transmit a signal at block 530 to an external device that is an inflatable bladder (e.g., an inflatable bladder of a pillow or mattress), which can cause the inflatable bladder to inflate and urge the user into a side-sleeping position. As other examples, transmitting a signal to an external device at block 530 can include transmitting a signal to increase or decrease a firmness of a mattress, increase or decrease an ambient temperature, raise or lower an ambient light level, raise or lower an ambient sound level (e.g., a level of white noise or other sounds), or take other action.

While certain aspects and features of the present disclosure are presented with respect to use of an oral appliance such as a mandibular repositioning device, such aspects and features can be used with respect to nerve stimulation devices. Such nerve stimulation devices can be incorporated into the oral appliance (e.g., as described above with reference to block 528), although that need not always be the case. In some cases, a nerve stimulation device can be placed on or under the skin, such as at or near a nerve like the hypoglossal nerve (e.g., positioned such that the electrodes of the nerve stimulation device are located at or near the nerve). Thus, the foregoing description of, and following claims related to, a system and method comprising an oral appliance also applies to a nerve stimulation device.

In such cases where a nerve stimulation device is used as, in addition to, or instead of an oral appliance, process 500 can be used to determine adjustments associated with the nerve stimulation device and facilitate applying such adjustments. For example, the determined adjustment can be a type, level, and/or location of an output stimulus (e.g., an electrical stimulus supplied to elicit a response in a nerve or muscle). In one example, the determined adjustment comprise hypoglossal nerve stimulation by a nerve stimulation device implanted under the skin which emits a stimulation pulse synchronized with the patient's breathing and which results in stimulation of the genioglossus muscle such as to move the user's tongue forward so as to prevent or reverse an airway obstruction. Determining the adjustment can occur similar to block 504, but as applied to a nerve stimulation device.

Facilitating applying the determined adjustment can occur similar to block 520, but as applied to a nerve stimulation device. For example, the adjustment can be implemented manually (e.g., by a user, physician, or technician) or remotely.

Determining (and facilitating) adjustments to a nerve stimulation device can be especially useful when the sensor data (e.g., sensor data received at block 502) is supplied by a non-contact sensor comprising an active acoustic and/or passive acoustic sensor, such as acoustic sensor 141 described herein. In such cases, the nerve stimulation device can be accurately and intelligently controlled and/or adjusted using non-contact sensors that do not interfere with the user's sleep and do not interfere with the nerve stimulation device.

In an example using a nerve stimulation device instead of an oral appliance as described herein, an adjustment in a pre-sleep state might be an imperceptible stimulation intended to strengthen the muscles in the neck to reduce likelihood of apneas or other events. For example, a nerve stimulation device can generate a stimulus to strengthen the upper airway dilator muscles such as the genioglossus muscle, which is innervated by the hypoglossal nerve, and the tensor palatini. Like those using oral appliance, systems using nerve stimulation devices can also make use of and control external devices, such as an inflatable bladders; can determine adjustments based on determining physiological data, estimating an autonomic tone, leveraging one or more sensors housed in a smartphone or tablet; and can otherwise function as described herein with respect to oral appliances, as appropriate.

While the present disclosure has been described with reference to one or more particular embodiments or implementations, those skilled in the art will recognize that many changes may be made thereto without departing from the invention as defined in the claims.

## Claims

1. A system comprising:
an oral appliance (122) configured to be used by a user for treatment of a sleep condition;
one or more sensor (130);
an oral appliance receptacle (126) configured to receive the oral appliance (122) when not used by the user;
a control system (110) including one or more processors (112); and
a memory (114), the control system (110) coupled to the memory (114), the memory (114) having stored thereon machine readable instructions which, when executed on the one or more processors (112), causes the one or more processors (112) to perform operations including:
receiving sensor data from the one or more sensors (130);
automatically determining an adjustment associated with the oral appliance (122) based on the sensor data; and
facilitating applying the determined adjustment to the oral appliance (122) in response to automatically determining the adjustment, wherein facilitating applying the determined adjustment includes actuating one or more actuators (128) of the oral appliance receptacle (126) to apply the determined adjustment to the oral appliance (122).

2. The system of claim 1, wherein facilitating applying the determined adjustment includes:
(i) presenting a display of one or more adjustment parameters to facilitate manual adjustment of the oral appliance (122) based on the one or more adjustment parameters; and/or
(ii) transmitting an adjustment signal to the oral appliance (122); and/or
(iii) dynamically or asynchronously adjusting the oral appliance (122) based on the determined adjustment.

3. The system of claim 1 or claim 2, wherein the oral appliance is a mandibular repositioning device.

4. The system of claim 1, wherein facilitating applying the determined adjustment includes dynamically or asynchronously adjusting the oral appliance (122) based on the determined adjustment, and wherein the operations further comprise:
receiving additional sensor data from the one or more sensors (130), wherein the additional sensor data is associated with use of the oral appliance (122) after dynamically or asynchronously adjusting the oral appliance (122) based on the determined adjustment;
automatically determining an additional adjustment associated with the oral appliance (122) based on the additional sensor data; and
facilitating applying the determined additional adjustment to the oral appliance (122) in response to automatically determining the additional adjustment.

5. The system of any one of claims 1 to 4, wherein facilitating applying the determined adjustment includes transmitting an adjustment signal to the oral appliance (122) and/or dynamically or asynchronously adjusting the oral appliance (122) based on the determined adjustment, and wherein automatically determining the adjustment associated with the oral appliance (122) based on the sensor data includes:
(i) determining that the user is in a pre-sleep state, wherein the determined adjustment is a preset pre-sleep adjustment for the pre-sleep state; or
(ii) determining that the user is in a post-onset-of-sleep state, wherein the determined adjustment is a preset post-onset-of-sleep adjustment for the post-onset-of-sleep state; or
(iii) identifying an apnea event, wherein the determined adjustment is a preset post-apnea adjustment for use after detection of the apnea event; or
(iv) predicting a future apnea event, wherein the determined adjustment is a pre-apnea adjustment for avoiding or minimizing the future apnea event.

6. The system of any one of claims 1 to 5, wherein the oral appliance (122) includes an electrical stimulator for applying electrical stimulus to the user, and wherein:
(i) applying the determined adjustment includes activating the electrical stimulator;
(ii) applying the determined adjustment includes adjusting a setting of the electrical stimulator;
(iii) applying the determined adjustment includes activating the electrical stimulator and directing the electrical stimulus to a tongue muscle or tongue nerve of the user via one or more electrodes included in the electrical stimulator; or
(iv) applying the determined adjustment includes adjusting a setting of the electrical stimulator and directing the electrical stimulus to a tongue muscle or tongue nerve of the user via one or more electrodes included in the electrical stimulator.

7. The system of any one of claims 1 to 6, wherein the operations further comprise:
determining that the user is using the oral appliance (122) based on the received sensor data; and
transmitting a signal to an external device (171) in response to determining that the user is using the oral appliance (122), wherein the signal, when received by the external device (171), adjusts a setting of the external device (171).

8. The system of claim 7, wherein the external device (171) includes an inflatable bladder, and wherein adjustment of the setting of the external device (171) includes adjusting the inflatable bladder to urge the user into a desired sleeping position.

9. The system of any one of claims 1 to 8, wherein the operations further comprise:
(i) accessing historical sensor data associated with a previous use of the oral appliance (122) by the user, wherein automatically determining the adjustment is further based on historical sensor data;
(ii) receiving subjective feedback from the user; and
associating the subjective feedback with the sensor data,
wherein automatically determining the adjustment is further based on the subjective feedback associated with the sensor data; and/or
(iii) determining a sleep stage based on the sensor data, wherein automatically determining the adjustment comprises using the determined sleep stage.

10. The system of any of claims 1 to 9, wherein automatically determining the adjustment includes:
(i) determining physiological data from the sensor data, wherein the physiological data comprises a breathing rate, a heart rate, or a blood oxygenation level;
identifying a desired change in the physiological data; and
determining the adjustment based on the desired change in the physiological data; and/or
(ii) estimating an autonomic tone of the user based on the sensor data;
identifying a desired change in the autonomic tone; and
determining the adjustment based on the desired change in the autonomic tone.

11. The system of any one of claims 1 to 10, wherein actuating the one or more actuators (128) of the oral appliance receptacle (126) changes a position of an upper tray of the oral appliance (122) relative to a lower tray of the oral appliance (122).

12. The system of any one of claims 1 to 10, wherein the one or more sensors (130) includes one or more sensors in the oral appliance (122), one or more sensors in the oral appliance receptacle (126), one or more sensors in a user device (170), one or more sensors in an external device (171), one or more sensors in the control system (110), one or more sensors positioned on or in contact with a portion of the user, one or more sensors worn by the user, one or more non-contact sensors spaced apart from the user, or any combination thereof.

13. The system of claim 12, wherein:
(i) the one or more sensors in the oral appliance (122) are configured to detect that the oral appliance (122) has been received by the oral appliance receptacle (122); and/or
(ii) the one or more sensors in the oral appliance receptacle (126) are configured to detect that the oral appliance (122) has been received by the oral appliance receptacle (122).

14. The system of any of claims 1 to 13, wherein the sleep condition is a sleep disordered breathing condition; or wherein the sleep condition is a sleep disordered breathing condition and the sleep disordered breathing condition is obstructive sleep apnea.

15. The system of any of claims 1 to 14, wherein the operations further comprise:
accessing at least one historical determined adjustment associated with the oral appliance (122) based on historical sensor data from a previous use of the oral appliance (122); and
determining effectiveness of the at least one historical determined adjustment based on at least one of the sensor data and historical sensor data,
wherein automatically determining the adjustment is further based on the determined effectiveness of the historical determined adjustment.

## Patentansprüche

1. System, umfassend:
eine orale Vorrichtung (122), die dazu ausgestaltet ist, von einem Benutzer zur Behandlung eines Schlafzustands verwendet zu werden;
einen oder mehrere Sensoren (130);
eine orale Vorrichtungsaufnahme (126), die dazu ausgestaltet ist, die orale Vorrichtung (122) aufzunehmen, wenn sie von dem Benutzer nicht verwendet wird;
ein Steuersystem (110) das einen oder mehrere Prozessoren (112) beinhaltet; und
einen Speicher (114), wobei das Steuersystem (110) mit dem Speicher (114) gekoppelt ist, wobei in dem Speicher (114) maschinenlesbare Anweisungen gespeichert sind, die, wenn sie auf dem einen oder den mehreren Prozessoren (112) ausgeführt werden, den einen oder die mehreren Prozessoren (112) veranlassen, Operationen durchzuführen, die Folgendes beinhalten:
Empfangen von Sensordaten von dem einen oder den mehreren Sensoren (130);
automatisches Bestimmen einer Einstellung, die der oralen Vorrichtung (122) zugeordnet ist, basierend auf den Sensordaten; und
Ermöglichen der Anwendung der bestimmten Einstellung auf die orale Vorrichtung (122) in Antwort auf automatisches Bestimmen der Einstellung, wobei Ermöglichen der Anwendung der bestimmten Einstellung Betätigen einer oder mehrerer Betätigungsvorrichtungen (128) der oralen Vorrichtungsaufnahme (126) beinhaltet, um die bestimmte Einstellung auf die orale Vorrichtung (122) anzuwenden.

2. System nach Anspruch 1, wobei Ermöglichen der Anwendung der bestimmten Einstellung Folgendes beinhaltet:
(i) Präsentieren einer Anzeige von einem oder mehreren Einstellungsparametern, um die manuelle Einstellung der oralen Vorrichtung (122) basierend auf dem einen oder den mehreren Einstellungsparameter zu ermöglichen; und/oder
(ii) Übertragen eines Einstellungssignals an die orale Vorrichtung (122); und/oder
(iii) dynamisches oder asynchrones Einstellen der oralen Vorrichtung (122) basierend auf der bestimmten Einstellung.

3. System nach Anspruch 1 oder Anspruch 2, wobei die orale Vorrichtung eine Einrichtung zur Repositionierung des Unterkiefers ist.

4. System nach Anspruch 1, wobei Ermöglichen der Anwendung der bestimmten Einstellung dynamisches oder asynchrones Einstellen der oralen Vorrichtung (122) basierend auf der bestimmten Einstellung beinhaltet, und wobei die Operationen ferner Folgendes umfassen:
Empfangen von zusätzlichen Sensordaten von dem einen oder den mehreren Sensoren (130), wobei die zusätzlichen Sensordaten der Verwendung der oralen Vorrichtung (122) nach dynamischem oder asynchronem Einstellen der oralen Vorrichtung (122) basierend auf der bestimmten Einstellung zugeordnet sind;
automatisches Bestimmen einer zusätzlichen Einstellung, die der oralen Vorrichtung (122) zugeordnet ist, basierend auf den zusätzlichen Sensordaten; und
Ermöglichen der Anwendung der bestimmten zusätzlichen Einstellung auf die orale Vorrichtung (122) in Antwort auf automatisches Bestimmen der zusätzlichen Einstellung.

5. System nach einem der Ansprüche 1 bis 4, wobei Ermöglichen der Anwendung der bestimmten Einstellung Übertragen eines Einstellungssignals an die orale Vorrichtung (122) und/oder dynamisches oder asynchrones Einstellen der oralen Vorrichtung (122) basierend auf der bestimmten Einstellung beinhaltet, und wobei automatisches Bestimmen der Einstellung, die der oralen Vorrichtung (122) zugeordnet ist, basierend auf den Sensordaten Folgendes beinhaltet:
(i) Bestimmen, dass der Benutzer in einem Vor-Schlafzustand ist, wobei die bestimmte Einstellung eine voreingestellte Vor-Schlaf-Einstellung für den Vor-Schlafzustand ist; oder
(ii) Bestimmen, dass der Benutzer in einem Zustand nach Einsetzen des Schlafs ist, wobei die bestimmte Einstellung eine voreingestellte Einstellung des Zustands nach Einsetzen des Schlafs für den Zustand nach Einsetzen des Schlafs ist; oder
(iii) Identifizieren eines Apnoe-Ereignisses, wobei die bestimmte Einstellung eine voreingestellte Nach-Apnoe-Einstellung zur Verwendung nach Erkennung des Apnoe-Ereignisses ist; oder
(iv) Vorhersagen eines zukünftigen Apnoe-Ereignisses, wobei die bestimmte Einstellung eine Vor-Apnoe-Einstellung zur Verhinderung oder Minimierung des zukünftigen Apnoe-Ereignisses ist.

6. System nach einem der Ansprüche 1 bis 5, wobei die orale Vorrichtung (122) einen elektrischen Stimulator zum Anwenden eines elektrischen Stimulus an den Benutzer beinhaltet, und wobei:
(i) Anwenden der bestimmten Einstellung Aktivieren des elektrischen Stimulators beinhaltet;
(ii) Anwenden der bestimmten Einstellung Einstellen einer Konfiguration des elektrischen Stimulators beinhaltet;
(iii) Anwenden der bestimmten Einstellung Aktivieren des elektrischen Stimulators und Leiten des elektrischen Stimulus zu einem Zungenmuskel oder Zungennerv des Benutzers über eine oder mehrere Elektroden, die in dem elektrischen Stimulator beinhaltet sind, beinhaltet; oder
(iv) Anwenden der bestimmten Einstellung Einstellen einer Konfiguration des elektrischen Stimulators und Leiten des elektrischen Stimulus zu einem Zungenmuskel oder Zungennerv des Benutzers über eine oder mehrere Elektroden, die in dem elektrischen Stimulator beinhaltet sind, beinhaltet.

7. System nach einem der Ansprüche 1 bis 6, wobei die Operationen ferner Folgendes umfassen:
Bestimmen, dass der Benutzer die orale Vorrichtung (122) verwendet, basierend auf den empfangenen Sensordaten; und
Übertragen eines Signals an eine externe Einrichtung (171) in Antwort auf Bestimmen, dass der Benutzer die orale Vorrichtung (122) verwendet, wobei das Signal, wenn es von der externen Einrichtung (171) empfangen wird, eine Konfiguration der externen Einrichtung (171) einstellt.

8. System nach Anspruch 7, wobei die externe Einrichtung (171) eine aufblasbare Blase beinhaltet, und wobei Einstellen der Konfiguration der externen Einrichtung (171) Einstellen der aufblasbaren Blase so beinhaltet, dass sie den Benutzer in eine gewünschte Schlafposition drängt.

9. System nach einem der Ansprüche 1 bis 8, wobei die Operationen ferner Folgendes umfassen:
(i) Zugreifen auf historische Sensordaten, die einer vorhergehenden Verwendung der oralen Vorrichtung (122) durch den Benutzer zugeordnet sind, wobei automatisches Bestimmen der Einstellung ferner auf den historischen Sensordaten basiert;
(ii) Empfangen einer subjektiven Rückmeldung von dem Benutzer; und Zuordnen der subjektiven Rückmeldung zu den Sensordaten ,
wobei automatisches Bestimmen der Einstellung ferner auf der subjektiven Rückmeldung, die den Sensordaten zugeordnet ist, basiert; und/oder
(iii) Bestimmen einer Schlafphase basierend auf den Sensordaten, wobei automatisches Bestimmen der Einstellung Verwenden der bestimmten Schlafphase umfasst.

10. System nach einem der Ansprüche 1 bis 9, wobei automatisches Bestimmen der Einstellung Folgendes beinhaltet:
(i) Bestimmen physiologischer Daten aus den Sensordaten, wobei die physiologischen Daten eine Atemfrequenz, eine Herzfrequenz oder einen Blutsauerstoffsättigungspegel umfassen;
Identifizieren einer gewünschten Veränderung in den physiologischen Daten; und
Bestimmen der Einstellung basierend auf der gewünschten Veränderung in den physiologischen Daten; und/oder
(ii) Schätzen eines autonomen Tonus des Benutzers basierend auf den Sensordaten;
Identifizieren einer gewünschten Veränderung im autonomen Tonus; und
Bestimmen der Einstellung basierend auf der gewünschten Veränderung im autonomen Tonus.

11. System nach einem der Ansprüche 1 bis 10, wobei Betätigen der einen oder mehreren Betätigungsvorrichtungen (128) der oralen Vorrichtungsaufnahme (126) eine Position eines oberen Fachs der oralen Vorrichtung (122) in Bezug auf ein unteres Fach der oralen Vorrichtung (122) verändert.

12. System nach einem der Ansprüche 1 bis 10, wobei der eine oder die mehreren Sensoren (130) einen oder mehrere Sensoren in der oralen Vorrichtung (122), einen oder mehrere Sensoren in der oralen Vorrichtungsaufnahme (126), einen oder mehrere Sensoren in einer Benutzereinrichtung (170), einen oder mehrere Sensoren in einer externen Einrichtung (171), einen oder mehrere Sensoren in dem Steuersystem (110), einen oder mehrere Sensoren, die auf oder in Kontakt mit einem Abschnitt des Benutzer positioniert sind, einen oder mehrere Sensoren, die von dem Benutzer getragen werden, einen oder mehrere kontaktiere Sensoren, die vom Benutzer beabstandet sind, oder eine beliebige Kombination daraus beinhalten.

13. System nach Anspruch 12, wobei:
(i) der eine oder die mehreren Sensoren in der oralen Vorrichtung (122) dazu ausgestaltet sind, zu erkennen, dass die orale Vorrichtung (122) durch die orale Vorrichtungsaufnahme (122) aufgenommen wurde; und/oder
(ii) der eine oder die mehreren Sensoren in der oralen Vorrichtungsaufnahme (126) dazu ausgestaltet sind, zu erkennen, dass die orale Vorrichtung (122) durch die orale Vorrichtungsaufnahme (122) aufgenommen wurde.

14. System nach einem der Ansprüche 1 bis 13, wobei der Schlafzustand ein Schlafzustand mit gestörter Atmung ist; oder wobei der Schlafzustand ein Schlafzustand mit gestörter Atmung ist und der Schlafzustand mit gestörter Atmung eine obstruktive Schlafapnoe ist.

15. System nach einem der Ansprüche 1 bis 14, wobei die Operationen ferner Folgendes umfassen:
Zugreifen auf zumindest eine historische bestimmte Einstellung, die der oralen Vorrichtung (122) zugeordnet ist, basierend auf historischen Sensordaten von einer vorhergehenden Verwendung der oralen Vorrichtung (122); und
Bestimmen von Wirksamkeit der zumindest einen historischen bestimmten Einstellung basierend zumindest auf einem der Sensordaten und der historischen Sensordaten,
wobei automatisches Bestimmen der Einstellung ferner auf der bestimmten Wirksamkeit der historischen bestimmten Einstellung basiert.

## Revendications

1. Système comprenant :
un appareil buccal (122) configuré pour être utilisé par un utilisateur pour le traitement d'une condition du sommeil ;
un ou plusieurs capteurs (130) ;
un récipient d'appareil buccal (126) configuré pour recevoir l'appareil buccal (122) lorsqu'il n'est pas utilisé par l'utilisateur;
un système de commande (110) incluant un ou plusieurs processeurs (112) ; et
une mémoire (114), le système de commande (110) étant couplé à la mémoire (114), la mémoire (114) présentant des instructions lisibles par une machine (114), stockées sur celle-ci qui, lorsqu'elles sont exécutées sur le un ou plusieurs processeurs (112) amènent le un ou plusieurs processeurs (112) à réaliser des opérations incluant :
la réception de données de capteur des un ou plusieurs capteurs (130) ;
la détermination automatique d'un ajustement associé à l'appareil buccal (122) sur la base des données de capteur ; et
la facilitation de l'application de l'ajustement déterminé à l'appareil buccal (122) en réponse à la détermination automatique de l'ajustement, dans lequel la facilitation de l'application de l'ajustement déterminé inclut l'actionnement d'un ou plusieurs actionneurs (128) du réceptacle de l'appareil buccal (126) afin d'appliquer l'ajustement déterminé à l'appareil buccal (122).

2. Système selon la revendication 1, dans lequel la facilitation de l'application de l'ajustement déterminé inclut :
(i) la présentation d'un affichage d'un ou plusieurs paramètres d'ajustement afin de faciliter l'ajustement manuel de l'appareil buccal (122) sur la base des un ou plusieurs paramètres d'ajustement ; et/ou
(ii) la transmission d'un signal d'ajustement à l'appareil buccal (122) ; et/ou
(iii) l'ajustement dynamique ou asynchrone de l'appareil buccal (122) sur la base de l'ajustement déterminé.

3. Système selon la revendication 1 ou 2, dans lequel l'appareil buccal est un dispositif de repositionnement mandibulaire.

4. Système selon la revendication 1, dans lequel la facilitation de l'application de l'ajustement déterminé inclut l'ajustement dynamique ou asynchrone de l'appareil buccal (122) sur la base de l'ajustement déterminé, et dans lequel les opérations comprennent en outre :
la réception de données de capteur supplémentaires à partir des un ou plusieurs capteurs (130), dans lequel les données de capteur supplémentaires sont associées à l'utilisation de l'appareil buccal (122) après ajustement dynamique ou asynchrone de l'appareil buccal (122) sur la base de l'ajustement déterminé ;
la détermination automatique d'un ajustement supplémentaire associé à l'appareil buccal (122) sur la base des données de capteur supplémentaires ; et
la facilitation de l'application de l'ajustement supplémentaire déterminé à l'appareil buccal (122) en réponse à la détermination automatique de l'ajustement supplémentaire.

5. Système selon l'une quelconque des revendications 1 à 4, dans lequel la facilitation de l'application de l'ajustement déterminé inclut la transmission d'un signal d'ajustement à l'appareil buccal (122) et/ou l'ajustement dynamique ou asynchrone de l'appareil buccal (122) sur la base de l'ajustement déterminé, et dans lequel la détermination automatique de l'ajustement associé à l'appareil buccal (122) basé sur les données de capteur inclut :
(i) la détermination que l'utilisateur est en état de pré-sommeil, dans lequel l'ajustement déterminé est un ajustement de pré-sommeil prédéfini pour l'état de pré-sommeil ; ou
(ii) la détermination que l'utilisateur est dans un état de post-début de sommeil, dans lequel l'ajustement prédéterminé est un ajustement post-début de sommeil déterminé pour l'état de post-début de sommeil ; ou
(iii) l'identification d'un événement d'apnée, dans lequel l'ajustement déterminé est un ajustement post-apnée prédéterminé pour une utilisation après détection de l'événement d'apnée ; ou
(iv) la prédiction d'un événement d'apnée futur, dans lequel l'ajustement déterminé est un ajustement pré-apnée pour éviter ou minimiser l'événement d'apnée futur.

6. Système selon l'une quelconque des revendications 1 à 5, dans lequel l'appareil buccal (122) inclut un stimulateur électrique pour appliquer un stimulus électrique à l'utilisateur, et dans lequel :
(i) l'application de l'ajustement déterminé inclut l'activation du stimulateur électrique ;
(ii) l'application de l'ajustement déterminé inclut l'ajustement d'un paramètre du stimulateur électrique ;
(iii) l'application de l'ajustement déterminé inclut l'activation du stimulateur électrique et la direction du stimulus électrique vers un muscle de la langue ou un nerf de la langue de l'utilisateur via une ou plusieurs électrodes incluses dans le stimulateur électrique ; ou
(iv) l'application de l'ajustement déterminé inclut l'ajustement d'un réglage du stimulateur électrique et de la direction du stimulus électrique vers un muscle de la langue ou un nerf de la langue de l'utilisateur via une ou plusieurs électrodes incluses dans le stimulateur électrique.

7. Système selon l'une quelconque des revendications 1 à 6, dans lequel les opérations comprennent en outre :
la détermination que l'utilisateur utilise l'appareil buccal (122) sur la base des données de capteur reçues ; et
la transmission d'un signal à un dispositif externe (171) en réponse à la détermination que l'utilisateur utilise l'appareil buccal (122), dans lequel le signal, lorsqu'il est reçu par le dispositif externe (171), ajuste un réglage du dispositif externe (171).

8. Système selon la revendication 7, dans lequel le dispositif externe (171) inclut une vessie gonflable, et dans lequel l'ajustement du réglage du dispositif externe (171) inclut l'ajustement de la vessie gonflable pour pousser l'utiliser dans une position de sommeil souhaitée.

9. Système selon l'une quelconque des revendications 1 à 8, dans lequel les opérations comprennent en outre :
(i) l'accès à des données historiques du capteur associées à une utilisation précédente de l'appareil buccal (122) par l'utilisateur, dans lequel la détermination automatique de l'ajustement est en outre basée sur des données historiques du capteur ;
(ii) la réception d'une rétroaction subjective de l'utilisateur ; et l'association de la rétroaction subjective aux données du capteur,
dans lequel la détermination automatique de l'ajustement est en outre basée sur la rétroaction subjective associée aux données du capteur ; et/ou
(iii) la détermination d'un stade de sommeil sur la base des données du capteur, dans lequel la détermination automatique de l'ajustement comprend l'utilisation du stade de sommeil déterminé.

10. Système selon l'une quelconque des revendications 1 à 9, dans lequel la détermination automatique de l'ajustement inclut :
(i) la détermination de données physiologiques à partir des données du capteur, dans lequel les données physiologiques comprennent un rythme respiratoire, une fréquence cardiaque ou un niveau d'oxygénation du sang ;
l'identification d'un changement souhaité des données physiologiques ; et
la détermination de l'ajustement sur la base du changement souhaité des données physiologiques ; et/ou
(ii) l'estimation d'un tonus autonome de l'utilisateur basé sur les données du capteur ;
l'identification d'un changement souhaité du tonus autonome ; et
la détermination de l'ajustement sur la base du changement souhaité de tonus autonome.

11. Système selon l'une quelconque des revendications 1 à 10, dans lequel l'actionnement des un ou plusieurs actionneurs (128) du réceptacle de l'appareil buccal (126) change une position du plateau supérieur de l'appareil buccal (122) par rapport à un plateau inférieur de l'appareil buccal (122).

12. Système selon l'une quelconque des revendications 1 à 10, dans lequel les un ou plusieurs capteurs (130) incluent un ou plusieurs capteurs dans l'appareil buccal (122), un ou plusieurs capteurs dans le réceptacle de l'appareil buccal (126), un ou plusieurs capteurs dans un dispositif utilisateur (170), un ou plusieurs capteurs dans un dispositif externe (171), un ou plusieurs capteurs dans le système de commande (110), un ou plusieurs capteurs positionnés sur ou en contact avec une partie de l'utilisateur, un ou plusieurs capteurs portés par l'utilisateur, un ou plusieurs capteurs sans contact espacés de l'utilisateur, ou toute combinaison de ceux-ci.

13. Système selon la revendication 12, dans lequel :
(i) les un ou plusieurs capteurs dans l'appareil buccal (122) sont configurés pour détecter que l'appareil buccal (122) a été reçu par le récipient de l'appareil buccal (122) ; et/ou
(ii) les un ou plusieurs capteurs dans le récipient de l'appareil buccal (126) sont configurés pour détecter que l'appareil buccal (122) a été reçu par le récipient de l'appareil buccal (122).

14. Système selon l'une quelconque des revendications 1 à 13, dans lequel la condition de sommeil est un trouble respiratoire du sommeil ; ou dans lequel la condition de sommeil est un trouble respiratoire du sommeil et le trouble respiratoire du sommeil est une apnée obstructive du sommeil.

15. Système selon l'une quelconque des revendications 1 à 14, dans lequel les opérations comprennent en outre :
l'accès à au moins un ajustement historique déterminé associé à l'appareil buccal (122) sur la base de données de capteur historiques d'une utilisation précédente de l'appareil buccal (122) ; et
la détermination de l'efficacité de l'au moins un ajustement historique déterminé sur la base d'au moins l'une des données du capteur et des données du capteur historiques,
dans lequel la détermination automatique de l'ajustement est en outre basée sur l'efficacité déterminée de l'ajustement historique déterminé.
